# EUROPEAN PATENT SPECIFICATION

(11) **EP 1 814 909 B1**
(45) Date of publication and mention of the grant of the patent: **24.08.2011**
(21) Application number: 05819055.4
(22) Date of filing: 18.11.2005
(51) Int. Cl.: C07K 14/47

(54) **USE OF AIMP2DX2 FOR THE DIAGNOSIS AND TREATMENT OF CANCER**
VERWENDUNG VON AIMP2DX2 ZUR DIAGNOSE UND BEHANDLUNG VON KREBS
UTILISATION DE AIMP2DX2 POUR DIAGNOSTIQUER ET TRAITER UN CANCER

(30) Priority: 24.11.2004 KR 20040097164; 10.05.2005 KR 20050039073
(43) Date of publication of application: 08.08.2007
(73) Proprietor: Neomics Co., Ltd., Seoul 151-742 (KR)
(72) Inventor: KIM, Sunghoon, F-1709, Tower Palace, Seoul 135-542 (KR)
(74) Representative: Lee, Nicholas John
(86) International application number: PCT/KR2005/003924
(87) International publication number: WO 2006/057500

(56) References cited:
- EP-A- 1 454 628
- KIM JIN YOUNG ET AL: "p38 is essential for the assembly and stability of macromolecular tRNA synthetase complex: Implications for its physiological significance" PROCEEDINGS OF THE NATIONAL ACADEMY OF SCIENCES OF THE UNITED STATES OF AMERICA, vol. 99, no. 12, 11 June 2002 (2002-06-11), pages 7912-7916, XP002479735 ISSN: 0027-8424
- OLGA C ET AL: 'The p38 subunit of the aminoacyl-tRNA synthetase complex is a Parkin substrate: linking protein biosynthesis and neurodegeneration.' HUMAN MOLECULAR GENETICS. vol. 12, no. 12, 15 June 2003, pages 1427 - 1437, XP008094790
- LEE S W ET AL: 'Aminoacyl-tRNA synthetase complexes: beyond translation.' JOURNAL OF CELL SCIENCE. vol. 117, no. 17, 01 August 2004, pages 3725 - 3734, XP008095224
- PARK S G ET AL: 'Dose-dependent Biphasic Activity of tRNA Synthetase-associating Factor, P43, in Angiogenesis.' J BIOL CHEM. vol. 277, no. 47, 22 November 2002, pages 45243 - 45248, XP002261380
- NORDIN B E ET AL: 'Plasticity of Recognition of the 3'-End of Mischarged tRNA by Class I Aminoacyl-tRNA Synthetases.' J BIOL CHEM. vol. 277, no. 23, 28 February 2002, pages 20510 - 20517, XP008095158

## Description

### FIELD OF THE INVENTION

The present invention relates to a variant of AIMP2 lacking exon 2, named as AIMP2DX2, which is specifically expressed in cancer cells, and its use for diagnosis and treatment of cancer.

### DESCRIPTION OF THE RELATED ART

The mortality rate due to cancer continues to increase worldwide. In spite of the collective studies about cancer, it is a major cause of mortality all over the world.

There is a need for a method capable of simply and quickly diagnosing cancers to improve effects of treatment methods such as surgery, radiotherapy, chemotherapy or conventional therapy.

It is required that markers specific to certain cancer are developed in order to diagnose cancer as well as apply an effective therapy for specific cancer. The cytotoxic therapy has been used broadly to treat cancers for last 50 years after it was used firstly as an anticancer agent. The therapy has serious side effects, for example, in which it acts nonspecifically on cells of another organ having a fast cell division rate comparative to cancer cells, resulting in strong toxicity. To overcome side effects and tolerance of such known anticancer agents, there have been many studies to develop anticancer medicaments specifically activating on tumor cells with cancer-specific-marker which is formed in tumorgenesis from normal cell to cancer. It is very important to find genes specific to cancer cell in cancer targeted therapy being represented as a method for minimizing a toxicity of anticancer agent.

Under such circumstances, the present inventors have made intensive research to develop a novel cancer-specific molecular species and as a result, found that a variant of AIMP2 lacking exon 2, AIMP2DX2 is specifically expressed in cancer cells and can be used as a marker for diagnosing cancer providing more reliable results. In addition, the present inventors have discovered that the antibody specific to AIMP2DX2 protein, or siRNA and antisense oligonucleotide specific to AIMP2DX2 mRNA may be applied for a specific treatment method for cancer cells.

### DESCRIPTION OF THE INVENTION

In a first aspect, the present invention provides a AIMP2DX2 protein comprising (a) a AIMP2 amino acid sequence in which the exon 2 region of the AIMP2 amino acid sequence is deleted, wherein AIMP2 has the sequence: or (b) the amino acid sequence of SEQ ID NO:2.

In a second aspect, the present invention provides a nucleic acid molecule comprising a nucleotide sequence encoding the AIMP2DX2 protein of the first aspect. The nucleic acid molecule may comprise the nucleotide sequence of SEQ ID NO:1.

In a third aspect, the present invention provides a recombinant vector, which comprises a nucleic acid molecule of the second aspect.

In a fourth aspect, the present invention provides a host cell which is transformed with the recombinant vector of the third aspect, wherein the cell is a non-human cell or is an isolated human cell that is not a human embryonic stem cell.

In a fifth aspect, the present invention provides a method for producing the AIMP2DX2 protein of the first aspect, comprising culturing the cell of the fourth aspect.

In a sixth aspect, the present invention provides an antibody specific to the AIMP2DX2 protein of the first aspect.

In a seventh aspect, the present invention provides a diagnostic kit for cancer, which comprises an antibody of the sixth aspect.

In an eighth aspect, the present invention provides a method for screening for AIMP2DX2 protein as a cancer marker, which comprises the steps of:
(a) providing a sample to be assayed; and
(b) detecting in said sample expression of a nucleic acid molecule of the second aspect, wherein detection of the expression of the nucleic acid molecule is indicative of cancer. The detecting in step (b) may be carried out by contacting said sample with an antibody of the sixth aspect. Alternatively, the detecting in step (b) may be carried out by an amplification reaction or a hybridization reaction. The amplification reaction may be carried out by RT-PCR (reverse transcription-polymerase chain reaction) using a primer capable of differentiating an mRNA of AIMP2DX2 of the first aspect from an mRNA of AIMP2. The hybridization reaction may be carried out by using a probe capable of differentiating an mRNA of AIMP2DX2 of the first aspect from an mRNA of AIMP2, wherein AIMP2 has the sequence:
The sequence of the primer may comprise SEQ ID Nos.5 and 6, 7 and 8 or 16 and 6. The sequence of the probe may comprise SEQ ID No. 8 or 16.

In a ninth aspect, the present invention provides a nucleic acid molecule comprising the nucleotide sequence of SEQ ID No.3. 4, 5, 6, 7, 8 or 16.

In a tenth aspect, the present invention provides a siRNA nucleic acid molecule specific to mRNA of the AIMP2DX2 protein of the first aspect, wherein said siRNAs nucleic acid molecule comprises sense and antisense RNA encoding for the nucleotide sequence of SEQ ID Nos. 9 and 10, SEQ ID Nos. 11 and 12 or SEQ ID Nos. 13 and 14.

In an eleventh aspect, the present invention provides an antisense oligonucleotide which is complementary to a junction region between exons 1 and 3 of an mRNA of the AIMP2DX2 protein of the first aspect.

In a twelfth aspect, the present invention provides a pharmaceutical composition for treating cancer comprising the siRNA nucleic acid molecule of the tenth aspect, or the antisense oligonucleotide of the eleventh aspect.

In a thirteenth aspect, the present invention provides the siRNAs nucleic acid molecule of the tenth aspect, or the antisense oligonucleotide of the eleventh aspect 16 for use in treating cancer.

In a fourteenth aspect, the present invention provides the use of the siRNA nucleic acid molecule of the tenth aspect, or the antisense oligonucleotide of the eleventh aspect in the manufacture of a medicament for treating cancer.

In a fifteenth aspect, the present invention provides a method of screening for an anticancer agent which inhibits the formation of a heterodimer between the AIMP2DX2 protein of the first aspect and the AIMP2 protein, comprising the steps of:
(a) contacting a test substance to a composition which comprises the AIMP2DX2 protein of the first aspect and the AIMP2 protein; and
(b) determining whether the test substance inhibits the heterodimer formation between the AIMP2DX2 protein of the first aspect and the AIMP2 protein,
   wherein the test substance to inhibit the heterodimer formation between the AIMP2DX2 protein of the first aspect and the AIMP2 protein is evaluated as an anticancer agent, and wherein AIMP2 has the sequence:

The method may be performed by a yeast-two-hybrid assay or in vitro pull-down assay.

In a sixteenth aspect, the present invention provides a method of screening for an anticancer agent which inhibits the expression of the gene encoding the AIMP2DX2 protein of the first aspect, or promotes the disruption of the AIMP2DX2 protein of the first aspect, comprising the steps of:
(a) contacting a test substance to cells which express the AIMP2DX2 gene; and
(b) determining whether the test substance inhibits the expression of the AIMP2DX2 gene or promotes the disruption of the AIMP2DX2 protein,
wherein the test substance to inhibit the expression of the AIMP2DX2 gene is evaluated as an anticancer agent. The method may be performed by RT-PCR or Western blot.

### BRIEF DESCRIPTION OF THE DRAWINGS

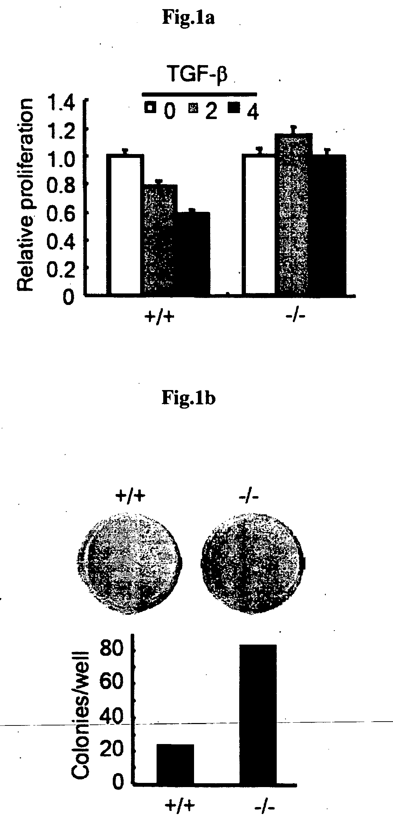

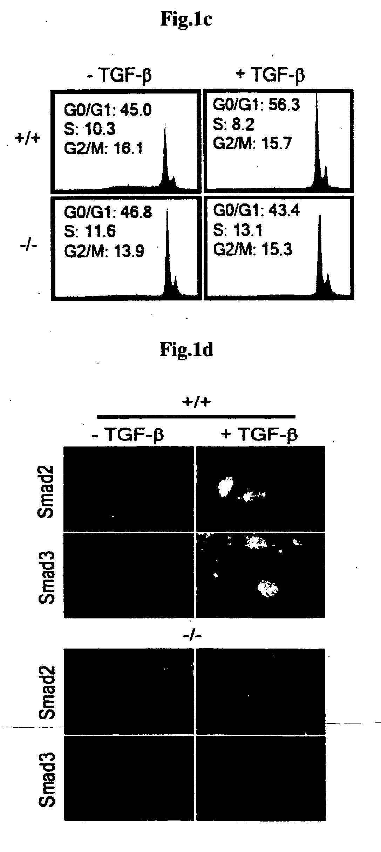

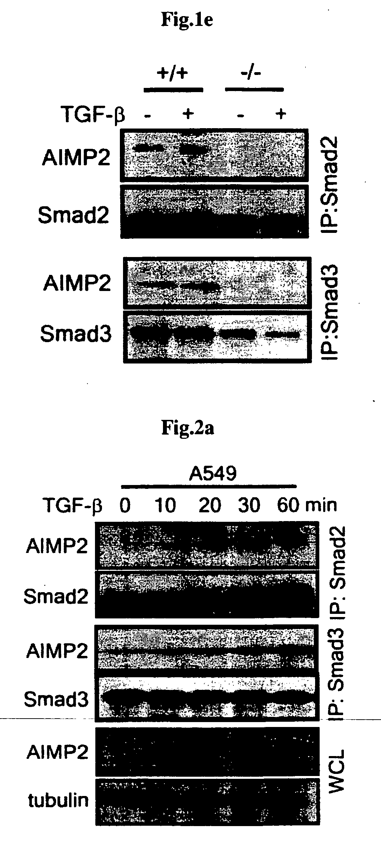

Figs. 1a-1e represent the functional importance of AIM2 in TGF-β signaling and its interaction with Smad2/3. AIMP2^{+/+} and AIMP2^{/-} MEFs were compared in the effect of TGF-β on cell proliferation (Fig. 1a), colony formation (Fig. 1b), cell cycle progression (Fig. 1c), and nuclear translocation of Smad2 and Smad3 (Fig. 1d). In Fig. 1a, the cells were incubated with the indicated concentrations (0, 2 and 4 ng/ml) of TGF-β1 for 6 hr. Thymidine incorporation in the untreated cells was taken as 1 and the values are the averages of four independent experiments. In Fig. 1b, AIMP2^{+/+} and AIMP2^{/-} MEFs (14.5 day) were cultivated in the presence of TGF-β (2 ng/ml) for 4 day, fixed with paraformaldehyde, and the colonies were visualized by Giemsa staining. In Fig. 1c, MEFs were treated with TGF-β for 24 hr, and the portion of G0/G1 phase cells was determined by flow cytometry. In Fig. 1d, MEFs were treated with TGF-β (2 ng/ml) for 1 hr. Smad2 and Smad3 were reacted with their specific antibodies and visualized by FITC-conjugated antibody (green). Nuclei were stained with PI (red). In Fig. 1e, the interactions of AIMP2 with Smad2 and Smad3 were tested by co-immunoprecipitation with antibodies against Smad2 and Smad3.

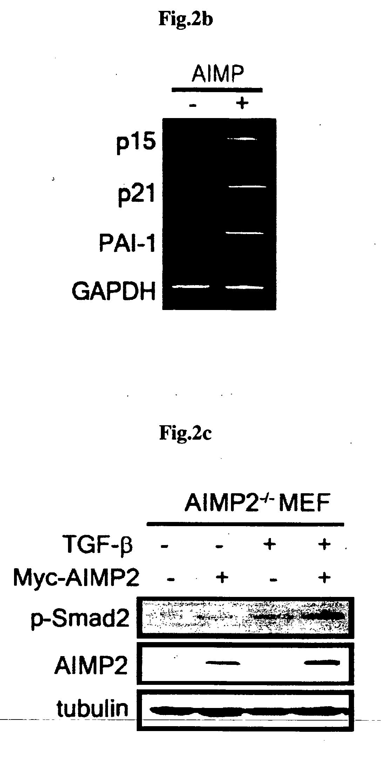

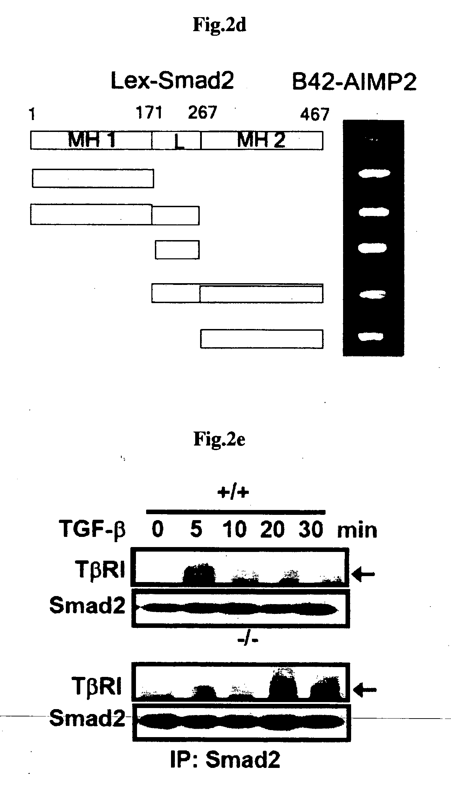

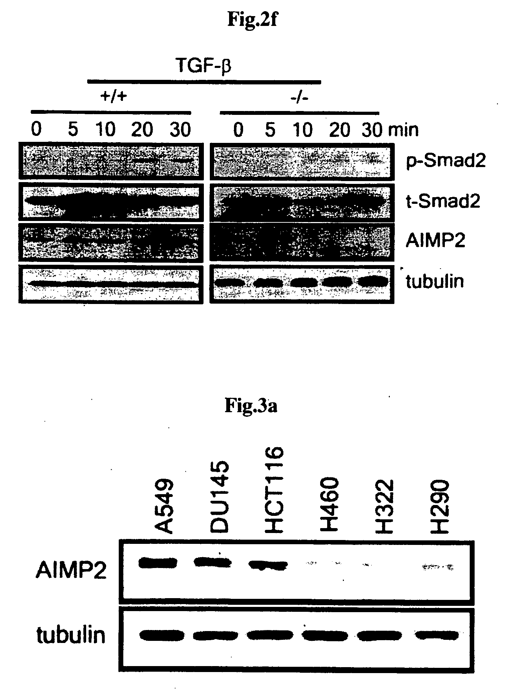

Figs. 2a-2f represent the working mechanism of AIMP2 in TGF-β signaling. In Fig. 2a, A549 cells were harvested at the indicated times after treatment of TGF-β (2 ng/ml) and the extracted proteins were immunoprecipitated with anti-Smad2 or Smad3 antibody, and coprecipitation of AIMP2 was determined with anti-AIMP2 antibody. WCL stands for the Western blots of the proteins of whole cell lysates. In Fig. 2b, the expression of the TGF-β target genes, p15, p21 and -PAI=I was determined by RT-PCR in the control and AIMP2transfected DU145. Fig. 2c demonstrates the effect of AIMP2 on the phosphorylation of Smad2 in AIMP2^{/-} MEFs. In Fig. 2d, the different domains of Smad2 (Mad-homology domain, MH1, MH2 and linker) were expressed as LexA fusion proteins and tested for the interaction with B42-fused AIMP2 by the blue colony formation on X-gal-containing yeast medium. In Fig. 2e, the TGF-β-dependent interaction of Smad2 with TGFβ receptor was determined by coimmunoprecipitation. MEFs were treated with TGF-β at 37°C and incubated at 8°C before immunoprecipitation. The association of TGF-β receptor with Smad2 was monitored by immunoblotting with anti-TβRI antibody. Fig. 2f shows the time course of the total Smad2, phosphorylated Smad2 (p-Smad2), and AIMP2 levels in AIMP2^{+/+} and AIMP2^{/-} MEFs after TGF- treatment.

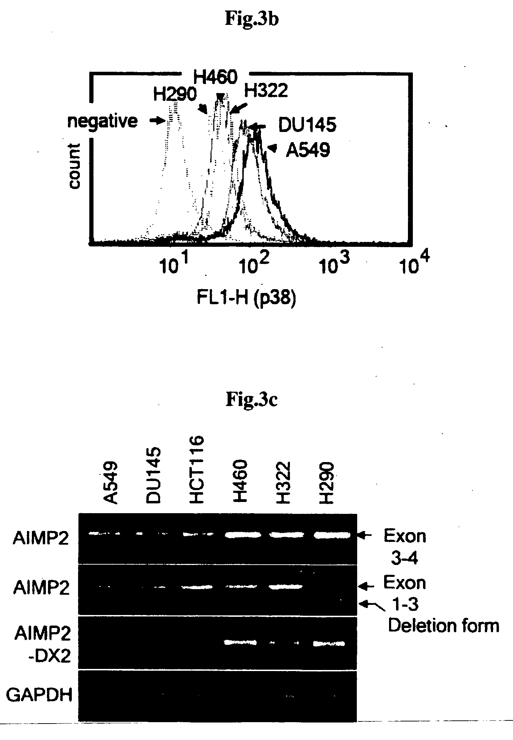

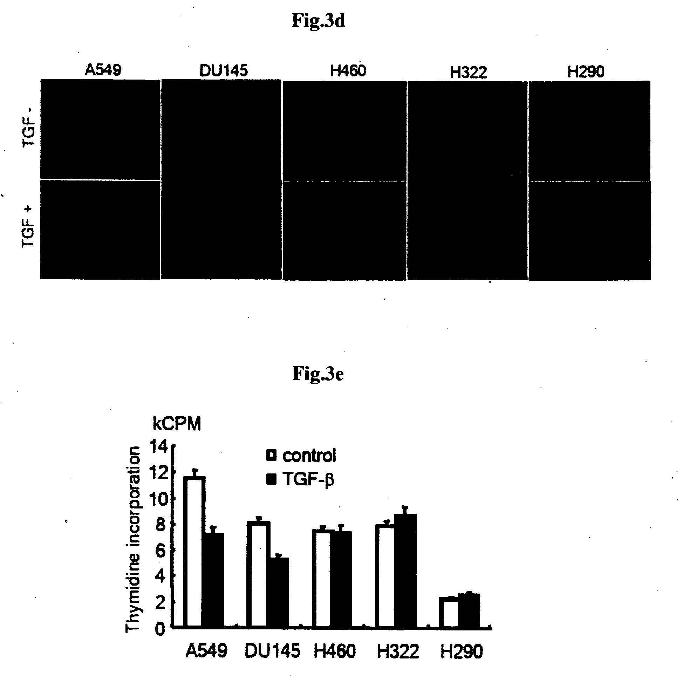

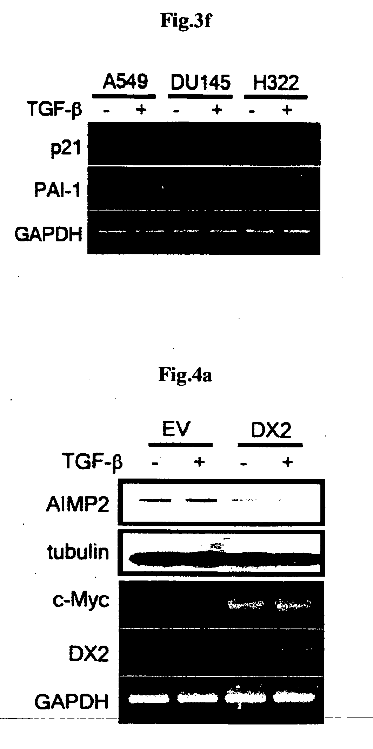

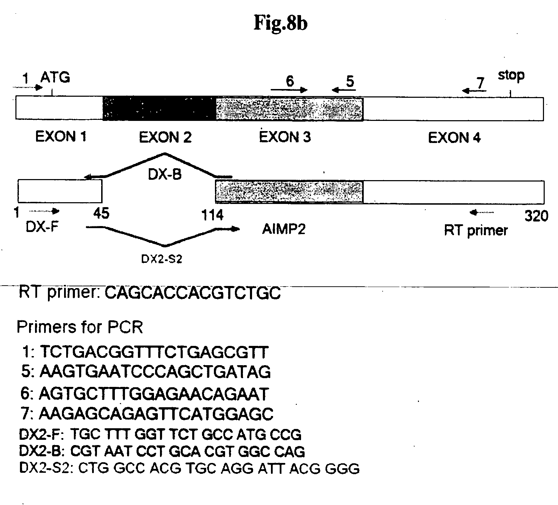

Figs. 3a-3f represents the differential expression of AIMP2 and generation of its exon 2-deletion form. The AIMP2 levels were compared in various cancer cell lines by Western blot analysis (Fig. 3a) and flow cytometry (Fig. 3b). A549, NCI-H460, -H322, and H290 are lung cancer cell lines while DU145 and HCT116 are prostate and colon cancer cell lines, respectively. In Fig. 3b, "negative" indicates DU145 treated only with secondary antibody. 2000 cells where analyzed for each cell line. In Fig. 3c, the AIMP2 transcript level was compared by RT-PCR with different primer pairs. The transcripts spanning exons 3-4 and 1-3 were generated by the primer pairs of 6/7 and 1/5 (see Fig. 8b), respectively. GAPDH is a loading control. Note the generation of a smaller AIMP2 transcript from the primer pair for the trasncript of exon -1-3. This smaller transcript is the alternative splicing form of AIMP2 lacking exon 2 (AIMP2DX2). This transcript was produced only from the low-AIMP2 cell lines by RT-PCR with the primer DX2-F and the other one specific to the junction sequence between exon 1 and 3 (DX2-B)(see Fig. 8b). In Fig. 3d, TGF-β-dependent induction and nuclear translocation of AIMP2 were examined by immunofluorescence staining after the incubation with TGF-β for 2 hr. In Fig. 3e, the effect of TGF-β was compared on the proliferation of the indicated cells by [³H] thymidine incorporation (n=9). In Fig. 3f, TGF-β-dependent induction of the target genes, p21 and PAI-1, was compared by RT-PCR after the incubation with TGF-β for 2 hr.

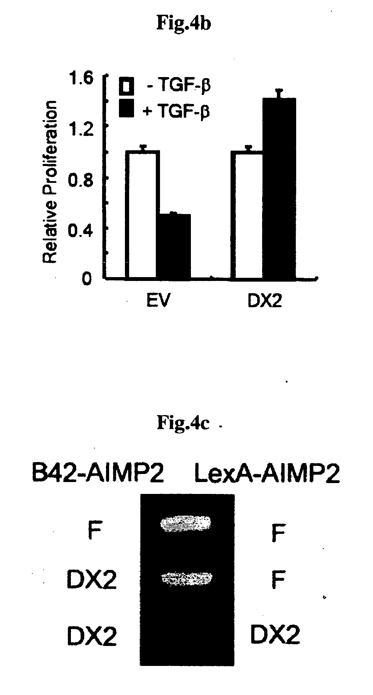

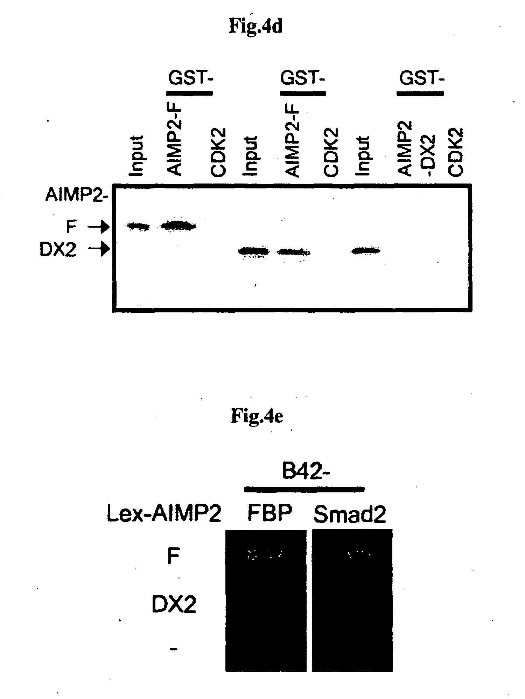

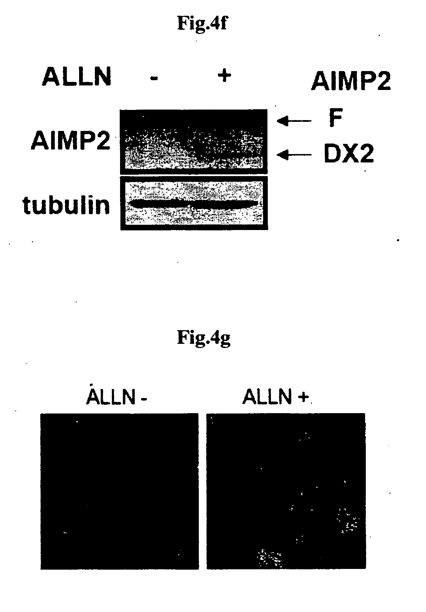

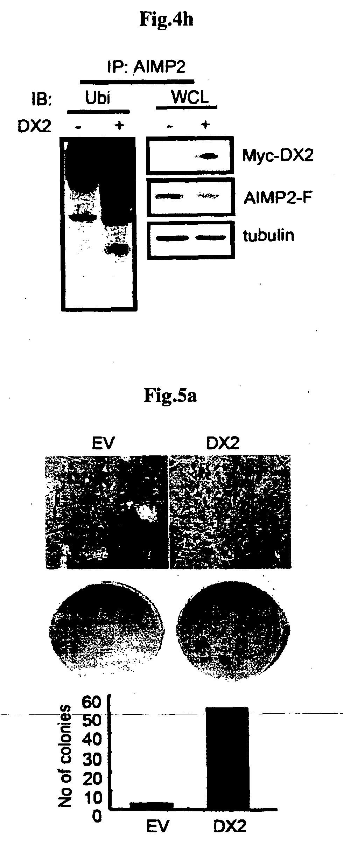

Figs 4a-4h demonstrates the Effect of AIMP2DX2 on the cellular stability of AIMP2. In Fig. 4a, DX2 was transfected into DU145 untreated or treated with TGF-β for 2 hr, and the AIMP2 level was determined by Western blotting. The expression of c-Myc, DX2 and GAPDH (control) was monitored by RT-PCR. In Fig. 4b, DX2 or empty vector was transfected into DU145, and its effect on the TGF-β-dependent cell growth inhibition was monitored by thymidine incorporation (n=4). In Fig. 4c, the interaction between AIMP2F and -DX2 was determined by yeast two hybrid assay as previously described (Rho, S.B. et al., PNAS. USA, 96:4488-93(1999)). In Fig. 4d, AIMP2F and -DX2 were synthesized by *in vitro* translation in the presence of [³⁵S] methionine, mixed with either GST-AIMP2F or -CDK2 (control), and precipitated with glutathione-Sepharose. The precipitated proteins were separated by SDS-PAGE and detected by autoradiography. In Fig. 4e, the interaction of AIMP2F or -DX2 with FUSE-binding protein (FBP; Kim, M.J. et al., Nat. Genet. 34:330-336:2003)) and Smad2 was determined by yeast two hybrid assay. In Fig. 4f, the effect of the proteasome inhibitor, ALLN (50 µM for 4 hr) on the levels of the full-length (F) and DX2 of AIMP2 was monitored in the DX2-generating H322 cells by Western blotting with anti-AIMP2 antibody. The DX2 form was confirmed by its comigration in gel with its *in vitro* synthesized counterpart. In Fig. 4g, the increase of AIMP2 by the treatment of ALLN (20 µM for 2 hr) was also shown by immunofluorescence staining with anti-AIMP2 antibody in H322 cells. In Fig. 4h, myc-tagged DX2 was transfected to DU145 that were treated with ALLN. Then, AIMP2 was immunoprecipitated with anti-AIMP2 antibody, and the ubiquitinated AIMP2 molecules were monitored by immunoblotting with anti-ubiquitin antibody (Ubi).

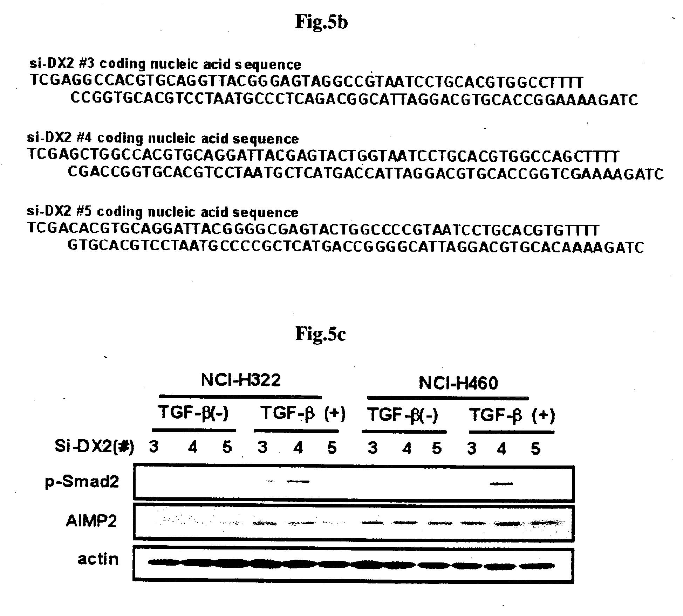

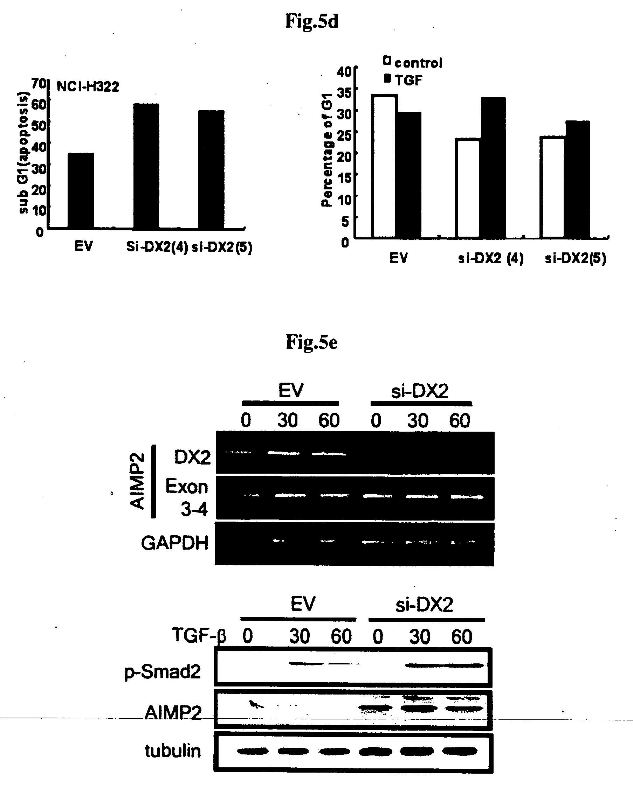

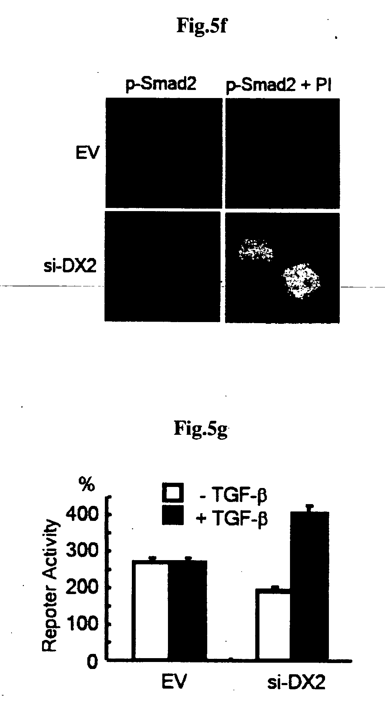

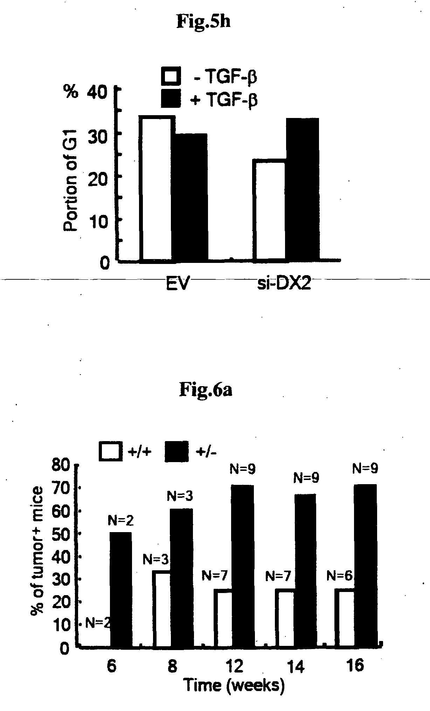

Figs. 5a to 5e represent the disruptive effect of AIMP2DX2 on cell growth control and TGF-β signaling. In Fig. 5a, DX2 (or empty vector) was transfected into MEFs and monitored its effect on cell growth. The cells and colonies were visualized by light microbe (top) and Giemsa staining (bottom), respectively. Fig 5b shows nucleotide sequences coding siRNAs specific to DX2. In Fig 5c, each vectors expressing 3 of siRNAs were constructed, transfected to H322 and H460 cell lines, and it's effect on AIMP2 level and smad2 phosphorylation were evaluated. In Fig 5d, siRNA of No4 or No5 were transfected to H322, and it's effect on cell death (left) and cell-cycle arrest (right) by the TGF-β were measured. In Fig. 5e, siRNA targeting AIMP2DX2 (si-DX2) was introduced into H322 and its suppressive effect on the DX2 transcript was determined by RT-PCR (top). si-DX2 did not affect the full-length AIMP2 transcript as shown by RT-PCR of the transcript for exon 3-4. The effect of si-DX2 on the phosphorylation of Smad2 and AIMP2 expression was also determined by Western blotting (bottom). The effect of si-DX2 on the restoration of the TGF-β signaling was also determined by immunofluorescence staining of p-Smad2 (Fig. 5f), TGF-β dependent reporter assay under 3TP promoter (Fig. 5g) and growth arrest (Fig. 5h) using H322 cells. In Fig. 5f, p-Smad2 and nuclei were stained with FITC-conjugated secondary antibody (green) and PI (red), respectively, 30 min after TGF-# treatment. Notice that p-Smad2 was increased and nuclear located by the transfection of si-DX2.

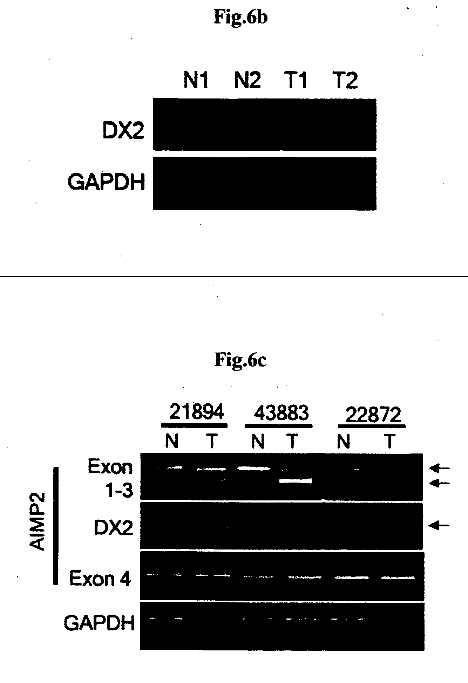

Figs. 6a to 6f represent the association of AIMP2 with lung cancer formation. In Fig. 6a, lung tumor formation was monitored at time interval after the intraperitoneal administration of benzo (α) pyrene into AIMP2^{+/-} and AIMP2^{+/-}mice. "N" stands for the number of the sacrificed mice. In Fig. 6b, total RNAs were isolated from the normal and tumor regions of a lung cancer patient, and subjected to RT-PCR with the DX2-specific primer. Normal and tumor tissues of the same patients (indicated by code number) were subjected to RT-PCR using anti-AIMP2 antibody (Fig. 6c).

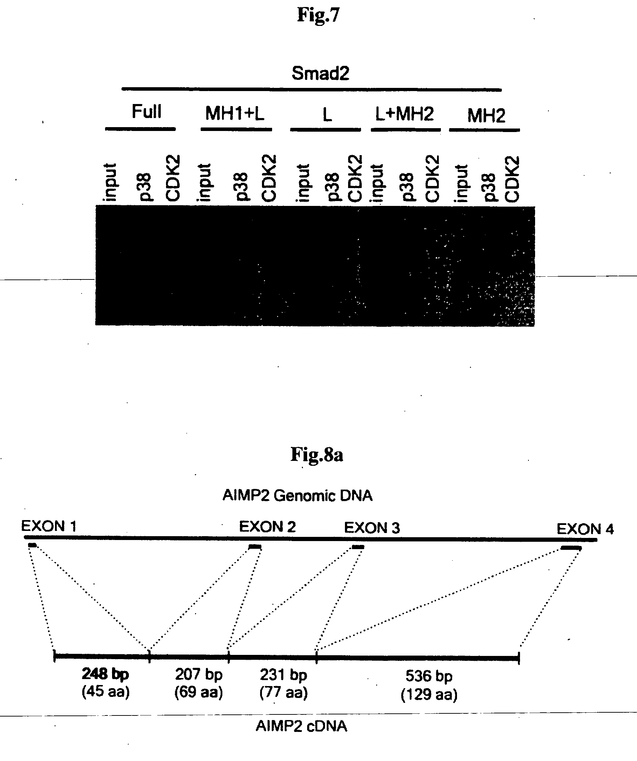

Fig. 7 represents the determination of the Smad2 domain involved in the interaction with AIMP2. We ligated the cDNAs encoding AIMP2 or CDK2 to the *EcoR*I and *Xho*I sites of pGEX4T-1 to express them in *E. coli* BL21 (DE3) as the GST-fusion proteins and purified them following the manufacturer's instruction. The different deletion fragments of Smad2 were synthesized by *in vitro* translation in the presence of [³⁵S] methionine using the TNT coupled translation kit (Promega). The GST fusion proteins bound to the glutathione Sepharose beads were incubated with the [³⁵S] methionine-labeled Smad2 fragments in the binding buffer of PBS buffer (pH 7.4) containing 0.5 mM EDTA, 0.5 mM phenylmethylsulfonylfluoride (PMSF), and 1% Trition X-100. The binding mixture was incubated overnight 4°C with rotation and washed four times with the binding buffer containing 0.5% Trition X-100. After addition of the SDS sample buffer, the bound proteins were eluted by boiling and separated by SDS gel electrophoresis. The presence of Smad2 fragments was determined by autoradiography.
Figs. 8a and 8b show the exon arrangement of human AIMP2 gene and the primer locations in AIMP2 cDNA. In Fig. 8a, the AIMP2 gene is composed of four exons encoding the polypeptides of the indicated size. Fig. 8b is the schematic representation for the locations of the primers used to generate the cDNAs spanning different regions of AIMP2 and their sequences.

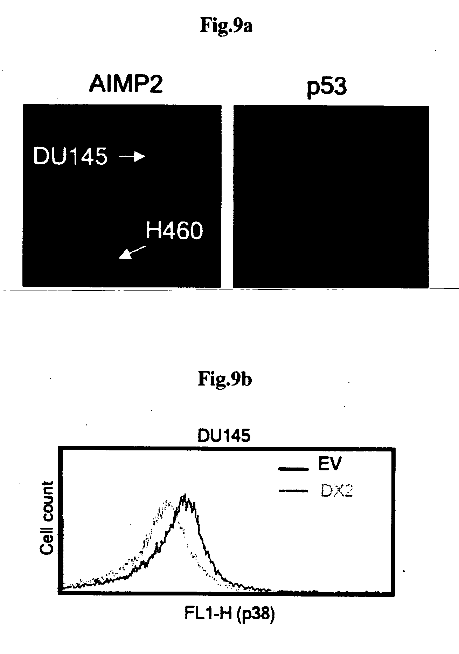

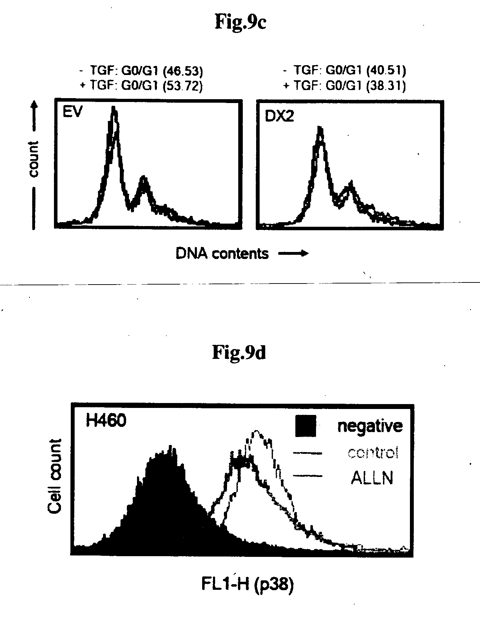

Figs. 9a to 9d demonstrate reduced expression of AIMP2, and generation of AIMP2DX2 in cancer cell lines. In Fig. 9a, to compare the AIMP2 levels between the DX2-positive and - negative cells, we cultured DU145 and H460 cells in one dish, and performed immunofluorescence staining with anti-AIMP2 antibody (green). The two cells lines were distinguished by immunofluorescence staining of p53 (red) since DU145 cells express p53 at high level due to its mutation, whereas H460 cells containing the wild type p53 maintain it at low level. The cells were treated with TGF-β for 2 hr, and fixed with methanol. In Fig. 9b, to address the effect of AIMP2DX2 on expression of AIMP2, the AIMP2 level was monitored by flow cytometry. 2 µg/ml of empty vector or DX2 were transfected into DU145 cells, and incubated for 24 hr. The cells were then fixed with 70% ethanol and reacted with anti-AIMP2 antibody, and subsequently FITC-conjugated secondary antibody. In Fig. 9c, the effect of AIMP2DX2 on the TGF-β-dependent cell cycle arrest was compared by flow cytometry. While the portion of the G0/G1 phase cells was increased in DU145 cells transfected with empty vector, but not in the DX2-transfected cells. The black and blues lines indicated the cells untreated and treated with TGF-β, respectively. In Fig. 9d, we compared the AIMP2 levels in H460 cells that were untreated (control) or treated with 10 µM ALLN for 2 hr. "Negative" indicates the cells incubated only with FITC-conjugated secondary antibody.

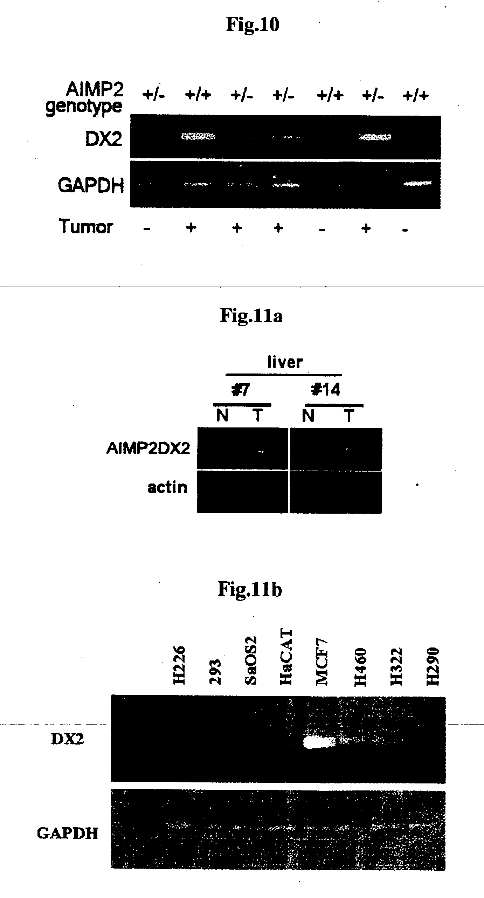

Figs. 10a and 10b show the generation of AIMP2DX2 and suppression of AIMP2 in lung cancer tissues. In Fig. 10a, lungs were isolated from the AIMP2^{+/+} and AIMP2^{+/-} mice injected with benzoypyrene, and RNAs were isolated from each lung for RT-PCR to determine the generation of AIMP2DX2. All of the lungs generating DX2 showed tumor formation in lung (marked +).
Figs. 11a and 11b represent the expression of AIMP2DX2 in various cancer tissues and cancer cell lines. In Fig. 11a, RT-PCR was performed to determine the expression of AIMP2DX2 in liver cancer tissues. Fig. 11b shows the expression of AIMP2DX2 in various cancer cell lines.

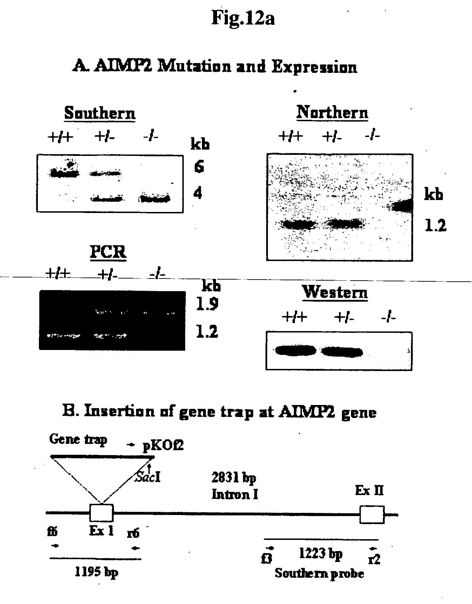

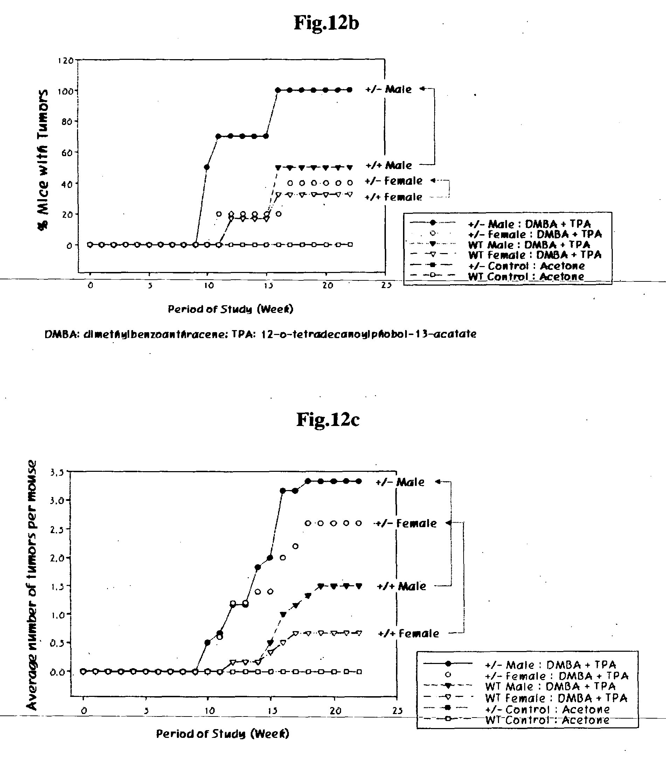

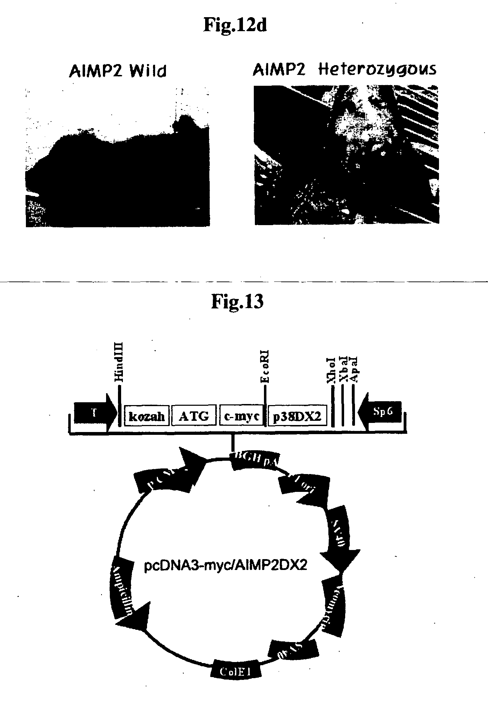

Fig.12 shows an indication of cancer in knocked-out (AIMP2+/-) mice comparing with a wild mouse normally expressing AIMP2. Fig. 12a, the mutation at the AIMP2 gene was determined by Southern blot and PCR analyses. The 1223bp PCR fragment was generated from intron I region of the mouse AIMP2 gene with the primers f3 and r2, and used as a probe for the hybridization of SacI fragments of mouse genomic DNA. The mixture of the primers, f6, r6 and pKOf2, was used for PCR analysis. The wild type and mutated allele generate about 1.2 and 1.9 kb PCR products, respectively. The mutational effect on the p AIMP2 expression was determined by Northern blotting with the AIMP2 cDNA probe and Western blotting with polyclonal rabbit antibody raised against the purified human AIMP2. The same amount of actin was detected in each lane of Northern blot (data not shown). +/+, +/- and -/- represent the wild type, heterozygous and homozygous mutant mice- The location of the gene trap insertion was determined by sequencing of the genomic DNA of the mutated allele. Fig 12b shows rate of tumor formation in AIMP2+/- mice and wild type mice. Fig. 12c shows average number of tumors per AIMP2+/- mouse and wild type mouse. Fig 12d shows papillomas developed in AIMP2+/- mice and wild type mice.
Fig. 13 shows the expression vector carrying the AIMP2DX2 gene. In Fig. 13, the abbreviations, pCMV, BGH pA, f1 ori, neomycin, ampicillin, SV40, SV40 pA, Co1E1, T7, and Sp6 denote human cytomegalovirus immediate-early promoter, bovine growth hormone polyadenylation signal, f1 replication origin, neomycin resistance gene, ampicillin resistance gene SV40 replication origin, SV40 polyadenylation signal, ColE1 replication origin, T7 viral promoter and Sp6 viral promoter, respectively.

### DETAILED DESCRIPTION OF THE INVENTION

AIMP2 (ARS-interacting multi-functional protein 2), named as AIMP2/JTV-1 or AIMP2 is one of proteins relating to the formation of aminoacyl-tRNA synthetase.
The present inventors elucidated that the genetic disruption of AIMP2 to induce overexpression of c-myc causes neonatal lethality in mice through overproliferation of alveolar epithelial cells and transforming growth factor-β (TGF-β) induces AIMP2 expression and promoted its translocation to nuclei for the downregulation of c-myc (M. J. Kim, et al., Nat. Genet. 34:330-336(2003)).
Following the previous research, the present inventors have revealed that AIMP2 is a novel tumor suppressor, playing a unique role in TGF-β signaling via interaction directly with Smad2/3. In addition, we have discovered that the aberrant variant of AIMP2 lacking exon II (AIMP2DX2) is specifically expressed in cancer cell lines and tissues. The existence of AIMP2 lacking exon II (AIMP2DX2) was verified by RT-PCR using combinations of AIMP2specific primers. When the primers were used to generate AIMP2 cDNA spanning exon 3 and 4, the decrease of AIMP2 transcript was not observed in the cells showing the reduced level of AIMP2 in Western blot analysis. When we used the primers generating the transcript from exon 1 to 3, we obtained not only the transcript of the expected size, but also a smaller one. Sequencing analysis of this small transcript revealed that it lacks exon 2 encoding 69 amino acid residues of AIMP2. RT-PCR analysis using the primer targeting to the junction sequence of exon 1 and 3 showed that the cell lines expressing lower AIMP2 level generated the smaller transcript, confirming the generation of AIMP2DX2 as AIMP2 variant.
Furthermore, the inventors observed that AIMP2 level was dramatically reduced regardless of TGF-β in the cell transformated with AIMP2DX2, demonstrating that the generation of AIMP2DX2 leads to loss of AIMP2 activity. In addition to this, the introduction of AIMP2DX2 into a cell elevated the expression of c-myc and relieved the growth arrest by TGF-β. We found that AIMP2DX2 forms an inactivated heterodimer with AIMP2 and is closely associated with tumorgenesis by inducing the decrease of AIMP2 level. In vivo studies provide additional evidence to verify that AIMP2DX2 is strongly related to lung cancer, liver cancer, skin cancer, breast cancer, renal cancer and osteosarcoma as well.

The AIMP2DX2 protein is a deletion variant of AIMP2 lacking exon 2. The amino acid sequence of the AIMP2 protein is found in several databases (312aa version: accession Nos. AAC50391.1 or GI:1215669; 320aa version: accession Nos. AAH13630.1, GI:15489023 and BC013630.1 available from GenBank) and publications (312aa version: Nicolaides, N.C., Kinzler,K.W. and Vogelstein,B. Analysis of the 5' region of PMS2 reveals heterogeneous transcripts and a novel overlapping gene, Genomics 29 (2):329-334(1995); 320 aa version: Generation and initial analysis of more than 15,000 full-length human and mouse cDNA sequences, Proc. Natl. Acad. Sci. U.S.A. 99(26): 16899-16903(2002)). The amino acid sequence of the AIMP2DX2 protein comprises, preferably, consists of that of AIMP2 lacking exon 2 region as afore-described known sequences. Korean Pat. Appln. No. 10-2004-0078035 discloses cancer therapy efficacy of the AIMP2 protein.

AIMP2DX2 protein includes exon 2-deleted variants of AIMP2 equivalents, for example, functional equivalents resulted from substitution, deletion, insertion or their combinations of AIMP2 that exhibit substantially identical activity to the wild type AIMP2, or functional derivatives with modifications to alter physical and/or biochemical properties of the wild type AIMP2 that exhibit substantially identical activity to the wild type AIMP2.
The deletion of exon 2 in AIMP2 as described herein means that the amino acid sequence spanning exon 2 region in AIMP2 (corresponding to amino acid 46-114) is partially or wholly deleted to generate a deletion variant of AIMP2 capable of forming a heterodimer with AIMP2 to inhibit normal function of AIMP2 and promote degradation of AIMP2. Accordingly, the AIMP2DX2 protein of this invention may include any variant of AIMP2 with whole or partial exon 2 deletion in which exon 1, 3 and/or 4 is natural or modified by amino acid substitution, deletion or insertion, so long as the variant is able to form a heterodimer with AIMP2 to inhibit normal function of AIMP2. Preferably, the AIMP2DX2 protein comprises a whole exon 2 deletion and intact exon 1, 3 and 4. More preferably, AIMP2DX2 protein comprises an amino acid sequence of SEQ ID NO:2.
The AIMP2DX2 protein may comprise its natural-occurring amino acid sequences and variants having modified sequences as well, so long as the variants retain activity of the AIMP2DX2 protein described above. The variants of the AIMP2DX2 protein refer to proteins having different sequences from its natural-occurring amino acid sequence prepared by deletion, insertion, non-conserved or conserved substitution or their combinations. The silent alteration of amino acid residues not to substantially impair protein activity is well known to one skilled in the art (H. Neurath, R.L. Hill, The Proteins, Academic Press, New York, 1979). Such amino acid alteration includes Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu and Asp/Gly, but is not limited thereto.
In addition, the AIMP2DX2 protein may comprise post-translational modifications such as phosphorylation, sulfation, acrylation, glycosylation, methylation and farnesylation.
The AIMP2DX2 protein and its variants may be obtained by the isolation from natural sources, synthesis (Merrifleld, J, Amer. Chem. Soc.. 85:2149-2156(1963)) or recombinant DNA technology (Joseph Sambrook, et al., Molecular Cloning, A Laboratory Manual, Cold Spring Harbor Laboratory Press, Cold Spring Harbor, N.Y.(2001)). Where a recombinant DNA technology is applied, host cells are transformed with an expression vector carrying a nucleic acid molecule encoding AIMP2DX2 and then cultured, followed by recovering AIMP2DX2 expressed.

As described previously, the AIMP2DX2 protein of the present invention is specifically expressed in a variety of cancer cells including breast cancer, lung cancer, liver cancer, blood cancer, skin cancer, renal cancer and osteosarcoma, demonstrating that the AIMP2DX2 protein can serve as a cancer diagnostic marker.

In another aspect of this invention, there is provided a nucleic acid molecule comprising a nucleotide sequence encoding the AIMP2DX2 protein described above. The above nucleotide sequence encoding AIMP2DX2 protein includes exon 2-deleted variant of AIMP2 equivalents, for example, functional equivalents resulted from substitution, deletion, insertion or their combinations of AIMP2 that exhibit substantially identical activity to the wild type AIMP2. The AIMP2DX2 protein of SEQ ID NO:2 is preferably encoded by the nucleic acid molecule comprising the nucleotide sequence of SEQ ID NO:1. The nucleic acid molecule of this invention may be single- or double-chain DNA (cDNA and gDNA) or single-chain RNA (mRNA).
The nucleic acid molecule encoding the AIMP2DX2 protein may be prepared by the isolation from natural sources, synthesis or recombinant DNA technology. The AIMP2DX2 nucleic acid molecule may be included in a suitable vector to provide the AIMP2DX2 protein.

In still another aspect of this invention, there is provided a recombinant vector which comprises a nucleic acid molecule comprising a nucleotide sequence encoding the AIMP2DX2 protein.
The term "recombinant vector" used herein refers to a genetic carrier to express a protein or RNA of interest in a suitable host cell, comprising a corresponding foreign sequence operably linked to a nucleic acid expression control sequence (such as a promoter, signal sequence and array of transcription factor binding sites).
The term "operably linked" used herein refers to functional linkage between a nucleic acid expression control sequence and a second nucleic acid sequence of interest, wherein the expression control sequence affects transcription and/or translation of the nucleic acid corresponding to the second sequence. The vector of this invention may be constructed according to conventional recombinant DNA technology in which site-specific DNA cleavage and ligation are performed using commercially available enzymes.
The vectors of the present invention include plasmid, cosmid, bacteriophage and viral vectors, but are not limited thereto. The vector may comprise expression control elements such as promoter, operator, starting and stopping codons, polyadenylation signal and enhancer as well as signal or leader sequences for membrane targeting or secretion. The promoter used in vectors may be constitutive or inducible one. Furthermore, the vector may carry a selection marker for selecting host cells harboring the vector and a replication origin.
The signal sequence in the vector includes, but not limited to, PhoA and OmpA signal sequences for *Escherichia* host cells, α-amylase and subtilicin signal sequences for *Bacillus* host cells, MFα and SUC2 signal sequences for yeast host cells, and insulin, α-interferon and antibody molecule signal sequences for animal host cells.

In further aspect of this invention, there is provided an isolated host cell which is transformed with the recombinant vector of this invention described above.
The transformation may be carried out according to any known approach for transforming nucleic acid molecules into organism, cell, tissue or organ, including electroporation, protoplasm fusion, CaPO₄ precipitation, CaCl₂ precipitation, agitation using silicon carbamide fiber, Agrobacterium-mediated transformation, PEG, dextran sulfate and lipofectamine, but not limited to. A suitable transformation method may be selected based on the type of host cells.
A suitable host cell is generally decided in considering the expression level and post-translation modification. Host cells include, but not limited to, prokaryotic cells such as *Escherichia coli, Bacillus subtilis, Streptomyces, Pseudomonas, Proteus mirabilis and Staphylococcus,* fungi (e.g., Aspergillus), yeast (e.g., *Pichia pastoris, Saccharomyces cerevisiae, Schizosaccharomyces* and *Neurospora crassa*), insect cells, plant cells and mammalian cells.

In still further aspect of this invention, there is provided a process for preparing the AIMP2DX2 protein by culturing host cells described previously.
The culturing of the host cells is carried out by conventional methods known to those skilled in the art under conditions suitable to express the AIMP2DX2 protein of interest.
The AIMP2DX2 protein expressed may be purified by conventional methods, for example, salting out (e.g., ammonium sulfate and sodium phosphate precipitation), solvent precipitation (e.g., protein fractionation precipitation using acetone or ethanol), dialysis, gel filtration, ion exchange, reverse-phase column chromatography, ultrafiltration or their combinations (Maniatis et al., Molecular Cloning: A Laboratory Manual, Cold Spring Harbor Laboratory, Cold Spring Harbor, N.Y. (1982): Sambrook et al., Molecular Cloning: A Laboratory Manual, 3rd Ed., Cold Spring Harbor Laboratory Press(2001); and Deutscher, M., Guide to Protein Purification Methods, Enzymology, vol. 182. Academic Press. Inc., San Diego, CA(1990)).

In another aspect of this invention, there is provided an antibody specific to the AIMP2DX2 protein.
The term "antibody" used herein means a protein molecule specifically directed toward an antigenic site. The antibody of this invention refers to antibodies to specifically recognize AIMP2DX2 and discriminate from AIMP2, including polyclonal and monoclonal antibodies.
Antibodies against the novel protein, AIMP2DX2, may be prepared in accordance with conventional technologies known to one skilled in the art.
Polyclonal antibodies may be prepared according to known processes in which the AIMP2DX2 protein as an immunogen is injected into animals and then antiserum is collected. Immunized animals include, but not limited to, goat, rabbit, sheep, monkey, horse, pig, cattle and dog.
Monoclonal antibodies may be prepared in accordance with a fusion method (Kohler and Milstein, European Journal of Immunology, 6 ; 511-519(1976)), a recombinant DNA method (USP 4,816,567) or a phage antibody library (Clackson et al, Nature, 352:624-628(1991); and Marks et al, J. Mol, Biol., 222:58, 1-597(1991)).
Antibodies against the AIMP2DX2 protein may be an intact immunoglobulin molecule which has two whole length of light chain and two whole length of heavy chain or its fragments containing antigen-binding site such as F(v), Fab, Fab' and F(ab')2.

The antibodies specific to AIMP2DX2 according to the present invention can be used to diagnose and treat cancer by suppressing the activity of AIMP2DX2. Where the antibodies are used as a therapeutic agent, they may be coupled to conventional therapeutic agents in direct or indirect (through a linker) manner.
The therapeutic agent coupled to the antibodies of this invention includes, but not limited to, radionuclide (e.g., 1311, 90Y, 105Rh, 47Sc, 67Cu, 212Bi, 211At, 67Ga, 125I, 186Re, 188Re, 177Lu, 153Sm, 123I and 111In), drug (e.g., methotrexate and adriamycin), lymphokine (interferon), toxin (ricin, abrin and diphtheria) and heterofunctional antibody that forms a complex with other antibody to posses a bi-functional binding capacity both to cancer cell and effector cell (e.g., T killer cell).
The antibody of this invention may be administered per se or in the form of a pharmaceutical composition.
The pharmaceutical composition comprising antibody may be formulated with a pharmaceutically acceptable carrier. The form of the pharmaceutical composition varies depending on the administration mode. Typically, the composition comprises one of surfactants for facilitating transmembrane delivery. Such surfactant includes steroid-derived compounds, cationic lipids such as N-[1-(2,3-dioleoyloxy)propyl]-N,N,N-trimethylammonium chloride (DOTMA), cholesterol hemisuccinate and phosphatidyl glycerol.

The pharmaceutical composition comprising the antibody of this invention is administered in a pharmaceutically effective amount to treat cancer or prevent cancer metastasis. The pharmaceutical composition may be administered in a single or multiple dosing regimen. The administration mode of the pharmaceutical composition includes parenteral, subcutaneous, intraperitoneal, intrapulmonary, intranasal, and local administration. Parenteral administration includes subcutaneous, intradermal, intramuscular, intravenous, intrabursa, intrasternal, intrathecal and intraperitoneal injection. The pharmaceutical composition is generally formulated in a pH range of 4-8 for antibody stability (chemical and physical stability) and safety. In addition, the pharmaceutical composition may be formulated in an oral dosing form. Typical dose is optimized using standard clinical techniques.
Furthermore, the antibody of this invention may be administered in a form of nucleic acid molecule to induce in vivo production of antibody (WO 96/07321).

In still another aspect of this invention, there is provided a diagnostic kit for cancer, which comprises an antibody specific to the AIMP2DX2 protein.
The cancer diagnosis kit of this invention may comprise antibody specific to AIMP2DX2 as well as general instruments and reagents for immunoassay including carrier, detectable signal-generating label, dissolving agent, washing agent, buffer and stabilizer. Where an enzyme is used as a label, its substrate and reaction quencher may be included. Non-limiting examples of carrier include soluble carriers, for example, physiologically acceptable buffer known in the art (e.g. PBS), insoluble carriers, for example, polystyrene, polyethylene, polypropylene, polyester, polyacrylonitrile, fluorine resin, cross-linked dextran, polysaccharides, polymers such as magnetic microparticle made of latex coated with a metal, paper, glass, metals, agarose and combinations thereof.
Non-limiting examples of the assay system useful in the cancer diagnosis kit of the present invention include ELISA plates, dip-stick devices, immunochromatography test strips and radial partition immunoassay devices and flow-through devices.

The term "cancer marker" used herein refers to a substance that provides information to evaluate cancer likelihood, occurrence or development by examining qualitatively or quantitatively its expression in tissues or cells. Preferably, the term means an organic biomolecule (e.g., protein, DNA and RNA) that is expressed in cancer cells in a different pattern from normal cells. The AIMP2DX2 gene or protein of this invention is specifically expressed in cancer cells but not in normal cells, permitting to accurately diagnose cancer. Preferably, the cancer diagnosis using AIMP2DX2 expression is performed in mRNA and/or protein level. The antibodies of the present invention are useful for diagnosing cancer such as lung cancer, liver cancer, breast cancer, skin cancer, renal cancer and osteosarcoma.

In further aspect of this invention, there is provided a method for screening AIMP2DX2 protein as a cancer-diagnosing marker, which comprises the steps of: (a) providing a sample to be assayed; and (b) detecting in the sample an expression of a nucleotide sequence encoding the AIMP2DX2 protein, wherein the detection of the expression of the nucleotide sequence encoding the AIMP2DX2 protein is indicative of cancer.
The term 'sample to be assayed' used in the present invention includes any biological sample such as tissue, cell, whole blood, serum, plasma, saliva, semen, urine, synovia and spinal fluid and may be pretreated for assay.
In the present invention, an expression of a nucleotide sequence encoding AIMP2DX2 protein in the sample may be carried out at protein or mRNA level. Where the method is performed to detect the AIMP2DX2 protein, antibodies to specifically recognize the AIMP2DX2 protein are used and the detection is carried out by contacting the sample with the antibody specific to the AIMP2DX2 protein and evaluating formation of antigen-antibody complex.

The term 'antigen-antibody complex' used in the present invention refers to a combination between AMIMP2DX2 protein and an antibody specific thereto in a biological sample. The evaluation on antigen-antibody complex formation may be carried out using immunohistochemical staining, radioimmuno assay (RIA), enzyme-linked immunosorbent assay (ELISA), Western blotting, immunoprecipitation assay, immunodiffusion assay, complement fixation assay, FACS and protein chip assay. The evaluation of antigen-antibody complex formation may be performed qualitatively or quantitatively, in particular, by measuring a signal from a detection label.
The label to generate a measurable signal for antigen-antibody complex formation includes, but is not limited to, enzyme, fluorophore, ligand, luminophore, microparticle, redox molecules and radioisotopes. The enzymatic label includes, but is not limited to, β-glucuronidase, β-D-glucosidase, β-D-galactosidase, urease, peroxidase, alkaline phosphatase, acetylcholinesterase, glucose oxidase, hexokinase, GDPase, RNase, luciferase, phosphofructokinase, phosphoenolpyruvate carboxylase, aspartate aminotransferase, phosphenolpyruvate decarboxylase, β-lactamase. The fluorescent label includes, but is not limited to, fluorescein, isothiocyanate, rhodamine, phycoerythrin, phycocyanin, allophysocyanin, o-phthalate and fluorescamine. The ligand serving as a label includes, but is not limited to, biotin derivatives. Non-limiting examples of the luminescent label include acridinium ester, luciferin and luciferase. Microparticles as label include colloidal gold and colored latex, but are not limited thereto. Redox molecules for labeling include ferrocene, lutenium complex compound, viologen, quinone, Ti ion, Cs ion, diimide, 1,4-benzoquinone, hydroquinone, K₄ W(CN)₈, [Os (bpy)₃]²⁺, [Ru (bpy)₃]²⁺ and [Mo (CN) ₈]⁴⁻, but not limited to. The radioisotopes include ³H, ¹⁴C, ³²P, ³⁵S, ³⁶Cl, ^{S1}Cr, ⁵⁷Co, ⁵⁸Co, ⁵⁹Fe, ⁹⁰Y, ¹²⁵I, ¹³¹I and ¹⁸⁶Re, but are not limited thereto.
Where the present method is performed to detect the AIMP2DX2 mRNA, the detection step may be carried out by an amplification reaction or a hybridization reaction well-known in the art.
The phrase "detection of the AIMP2DX2 mRNA" used herein is intended to refer to analyzing the existence or amount of the AIMP2DX2 mRNA as a cancer diagnosis marker in cells by use of a primer or probe specifically hybridized with the AIMP2DX2 mRNA.
The term "primer" used herein means an oligonucleotide having a free 3' hydroxyl group, whether occurring naturally as in a purified restriction digest or produced synthetically, which is capable of forming base pairs with a complementary template and acting as a point of initiation of synthesis when placed under conditions in which synthesis of a primer extension product which is complementary to a nucleic acid strand is induced. The term "probe" used herein refers to a linear oligomer of natural or modified monomers or linkages, including deoxyribonucleotides, ribonucleotides and the like, which is capable of specifically hybridizing with a target nucleotide sequence, whether occurring naturally or produced synthetically. The probe used in the present method may be prepared in the form of oligonucleotide probe, single-stranded DNA probe, double-stranded DNA probe and RNA probe. It may be labeled with biotin, FITC, rhodamine, DIG and radioisotopes. For example, the nucleotide sequence of SEQ ID No. 8 or 16 can be used as a probe.
The method to detect the AIMP2DX2 mRNA using either a primer or probe includes, but is not limited to, DNA sequencing, RT-PCR (reverse transcription-polymerase chain reaction), primer extension method (Nikiforov, T.T. et al., Nucl Acids Res 22, 4167-4175(1994)), oligonucleotide ligation analysis (OLA) (Nickerson, D.A. et al., Pro Nat Acad Sci USA, 87, 8923-8927(1990)), allele-specific PCR (Rust, S. et al., Nucl Acids Res, 6, 3623-3629(1993)), RNase mismatch cleavage (Myers R.M. et al., Science, 230, 1242-1246(1985)), single strand conformation polymorphism-(SSCP; Orita M. et al., Pro Nat Acad Sci USA, 86, 2766-2770(1989)), simultaneous analysis of SSCP and heteroduplex (Lee et al., Mol Cells, 5:668-672(1995)), denaturation gradient gel electrophoresis (DGGE; Cariello NF. et al., Am J Hum Genet, 42, 726-734 (1988)) and denaturing high performance liquid chromatography (D-HPLC, Underhill PA. et al., Genome Res, 7, 996-1005(1997)).
Preferably, the method by amplification reaction is carried out by RT-PCR using a primer capable of differentiating an mRNA of AIMP2DX2 from an mRNA of AIMP2. RT-PCR process suggested by P. Seeburg (1986) for RNA research involves PCR amplification of cDNA obtained from MRNA. reverse transcription. For amplification, a primer pair specifically annealed to AIMP2DX2 is used. Preferably, the primer is designed to generate two different sized bands in electrophoresis in which one is specific to the AIMP2 mRNA and the other to AIMP2DX2 mRNA. Alternatively, the primer is designed to generate only an electrophoresis band specific to AIMP2DX2 mRNA. The primer pair to generate two different sized bands for the AIMP2 RNA and AIMP2DX2 mRNA is prepared to amplify a region corresponding to exon 2. The nucleotide sequence of such primers is not limited; but is, most preferably, a primer set consisting of SEQ ID NOs:5 and 6. To observe only one electrophoresis band specific to AIMP2DX2 mRNA, one of primers is designed to comprise the junction sequence between C-terminal of exon 1 and N-terminal of exon 3 . In Examples described below, for RT-PCR the primer of SEQ ID NO:8 annealed to the junction sequence is used together with the primer of SEQ ID NO:7 or the primer of SEQ ID NO:16 annealed to the junction sequence is used together with the primer of SEQ ID NO:6. The RT-PCR analysis is convenient in the sense that cancer diagnosis is accomplished by observing the electrophoresis band pattern to evaluate expression of the AIMP2DX2 mRNA.
The symptoms of cancer including lung cancer, liver cancer, breast cancer, skin cancer, renal cancer and osteosarcoma can be diagnosed from significant differences of AIMP2DX2 expression between a sample and a control.

According to a preferable aspect of the present invention, there is provided an siRNA (small interfering RNA) molecule specific to an mRNA of AIMP2DX2.
The term "siRNA" used herein refers to a short RNA duplex to induce RNAi (RNA interference) phenomenon through mRNA cleavage. The siRNA consists of a sense RNA strand corresponding to a target mRNA and an antisense RNA strand complementary to a target mRNA. siRNAs to inhibit expression of a target gene provides effective gene knock-down method or gene therapy method.
The siRNA of this invention is not restricted to a RNA duplex of which two strands are completely paired and may comprise a non-paired portion such as mismatched portion with non-complementary bases and bulge with no opposite bases. The overall length of the siRNA may be 10-100 nucleotides, preferably, 15-80 nucleotides, and more preferably, 20-70 nucleotides. The siRNA may comprise either a blunt or cohesive end so long as it can silence expression of AIMP2DX2 due to the RNAi effect. The cohesive end may be prepared with a 3'-end overhanging structure or 5'-end overhanging structure. The overhanging bases are not limited in their length, for example, 1-8 nucleotides, preferably, 2-6 nucleotides. The overall length as described herein is expressed as the total of length of central double-stranded portion and terminal single-stranded overhanging portion. Furthermore, as long as AIMP2DX2 siRNA is able to maintain its gene silencing effect on the target gene, it may contain a low molecular weight RNA (which may be a natural RNA molecule such as tRNA, rRNA or viral RNA, or an artificial RNA molecule), for example, in the overhanging portion at its either end. It is not necessary that both ends of AIMP2DX2 siRNA have a cleavage structure. The AIMP2DX2 siRNA of this invention may comprise a stem-loop structure in which either end (head or tail) is connected via a linker. The length of the linker is not limited unless it impairs base pairing in the stem structure.
The term "specific" used herein is intended to express the inhibition of target gene expression with no influence on other genes. The siRNA of this invention is specific to the AIMP2DX2 gene.
It is preferred that the siRNA of this invention comprises a sense strand containing a corresponding sequence to a junction sequence between exons 1 and 3 and an antisense strand containing a complementary sequence.
The phrase "inhibition of gene expression" means that the level of mRNA and/or protein generated from the target gene is quenched or reduced, which is induced by RNA interference via occurrence of mRNA cleavage.
The siRNA of this invention may be synthesized in vitro and then introduced into cells via transfection. In addition, it may be transfected into cells in the form of an siRNA expression vector or a PCR-derived siRNA expression cassette. Suitable preparation and transfection methods may be determined in considering the experiment aim and target gene function.
The sequences and length of the siRNA are not limited as long as it is able to specifically suppress AIMP2DX2 mRNA. 3 illustrative siRNA expression vectors to silence AIMP2DX2 are found in the Examples described hereunder, suppressing cellular level of AIMP2DX2 and restoring AIMP2 function and TGF-β signal transduction.
The preferable siRNA of this invention comprises a corresponding sequence to the junction sequence between exons 1 and 3 of the AIMP2 DX2 mRNA. More preferably, the siRNA of this invention is a RNA duplex described as (i) No.3 siRNA consisting of two RNA molecules expressed from nucleotide sequences of SEQ ID NOs:9 and 10, (ii) No.4 siRNA consisting of two RNA molecules expressed from nucleotide sequences of SEQ ID NOs:11 and 12, or (iii) No.5 siRNA consisting of two RNA molecules expressed from nucleotide sequences of SEQ ID NOs:13 and 14. The siRNA consisting of two RNA molecules expressed from nucleotide sequences of SEQ ID NOs:11 and 12 is most preferred. The single or mixed type of siRNAs molecules may be used.

In a still further aspect of this invention, there is provided an antisense oligonucleotide which is complementary to a region of an mRNA of the AIMP2DX2 protein.
The term "antisense oligonucleotide" used herein is intended to refer to nucleic acids, preferably, DNA, RNA or its derivatives, that are complementary to the base sequences of a target mRNA, characterized in that they bind to the target mRNA and interfere with its translation to protein. The antisense oligonucleotide of this invention means DNA or RNA sequences complementary and binding to AIMP2DX2 mRNA, that are able to inhibit translation, translocation, maturation or other biological functions of AIMP2DX2 mRNA. The antisense nucleic acid is preferably 6-100, or preferably, 8-60, or more preferably, 10-40 nucleotides in length.
The antisense oligonucleotide may have at least one modification in its base, sugar or backbone for its higher inhibition efficacy (De Mesmaeker et al., Curr Opin Struct Biol., 5 (3) :343-55(1995)). The modified nucleic acid backbone comprises phosphorothioate, phosphotriester, methyl phosphonate, short chain alkyl or cycloalkyl intersugar linkages or short chain heteroatomic or heterocyclic intersugar linkages. The antisense oligonucleotide may also contain one or more substituted sugar moieties. The antisense nucleic acid may include one or more modified bases, for example, hypoxanthine, 6-methyladenine, 5-me pyrimidines (particularly, 5-methylcytosine), 5-hydroxymethylcytosine (HMC), glycosyl HMC and gentobiosyl HMC, 2-aminoadenine, 2-thiouracil, 2-thiothymine, 5-bromouracil, 5-hydroxymethyluracil, 8-azaguanine, 7-deazaguanine, N⁶(6-aminohexyl)adenine and 2,6-diaminopurine. Another modification of the oligonucleotides of the invention involves chemically linking to the oligonucleotide one or more moieties or conjugates which enhance the activity or cellular uptake of the oligonucleotide. Such moieties include but are not limited to lipid moieties such as a cholesterol moiety, a cholesteryl moiety, cholic acid, thioether, thiocholesterol, an aliphatic chain, phospholipid, polyamine, polyethylene glycol chain, adamantane acetic acid, palmityl moiety, octadecylamine, hexylamino-carbonyl-oxycholesterol moiety. Oligonucleotides comprising lipophilic moieties, and methods for preparing such oligonucleotides are known in the art, for example, U.S. Pat. Nos. 5,138,045, 5,218,105 and 5,459,255. The modifications described above enhance stability against nuclease degradation and increase affinity of the antisense oligonucleotide toward its target mRNA.
It is preferred that the antisense oligonucleotide specific to AIMP2DX2 comprises a complementary sequence to a junction region between exons 1 and 3 of AIMP2DX2 mRNA.

The antisense RNA molecule is conventionally synthesized in vitro and then transmitted to cells. In addition, it is intracellularly produced by transcription from foreign sequence. In vitro synthesis involves RNA polymerase I. In vivo transcription for preparing antisense RNA uses a vector having an origin of recognition region (MCS) in opposite orientation. The antisense RNA preferably comprises a translation stop codon for inhibiting translation to a peptide.

In another aspect of this invention, there is provided a pharmaceutical composition for treating cancer, which comprises (a) the antisense oligonucleotide or siRNA specific to AIMP2DX2 mRNA as an active ingredient; and (b) a pharmaceutically acceptable carrier.

Also described herein is a method for treating cancer in a patient, which comprises administrating into the patient a pharmaceutical composition comprising (a) the antisense oligonucleotide or siRNA specific to AIMP2DX2 mRNA as an active ingredient; and (b) a pharmaceutically acceptable carrier.
The pharmaceutical composition comprising at least one of AIMP2DX2 siRNAs or antisense oligonucleotides may contain an additional agent to suppress tumor cell proliferation and to facilitate the transduction of siRNA or antisense nucleic acid, for example, liposome (U.S. Pat. Nos. 4,897,355, 4,394,448, 4,235,871, 4,231,877, 4,224,179, 4,753,788, 4,673,567, 4,247,411 and 4,814,270), or lipophilic carrier including sterols such as cholesterol, cholate and deoxycholic acid. In addition, the siRNA or antisense nucleic acid may be conjugated to cell-adsorbing peptides such as peptide hormones, antigens and peptide toxins (Haralambid et al, WO 89/03849; Lebleu et al., EP 0263740).
Where the pharmaceutical composition is formulated for oral administration with a pharmaceutically acceptable carrier, it may contain binder, lubricant, disintegrator, diluent, solubilizer, dispersing agent, stabilizer, suspending agent, pigment and sweetner. Where the pharmaceutical composition is formulated for injection, it may contain buffer, preservative, analgesic, solubilizer, tonicity agent and stabilizer. For topical administration, the pharmaceutical composition may contain a substrate, diluent, lubricant and preservative. The formulation of the pharmaceutical composition may be prepared by formulating with the pharmaceutically acceptable carriers described above. For oral administration, the pharmaceutical composition may be in the form of a tablet, troche, capsule, elixir, suspension, syrup and water. The injectable composition may be formulated in unit dosage ample or multi dosage form.
The correct dosage of the pharmaceutical compositions of this invention comprising AIMP2DX2 siRNAs or antisense oligonucleotide will be varied according to the particular formulation, the mode of application, age, body weight and sex of the patient, diet, time of administration, condition of the patient, drug combinations, reaction sensitivities and severity of the disease. It is understood that the ordinary skilled physician will readily be able to determine and prescribe a correct dosage of the pharmaceutical compositions.
The administration mode of the pharmaceutical composition includes oral and parenteral such as subcutaneous, intradermal, intramuscular, intravenous, intrabursa, intrasternal, intrathecal and intraperitoneal injections.

In further aspect of this invention, there is provided a method of screening for an anticancer agent which inhibits the formation of a heterodimer between AIMP2DX2 protein of claim 1 and AIMP2 protein, comprising the steps of: (a) contacting a test substance to a composition which comprises AIMP2DX2 protein and AIMP2 protein; and (b) determining whether the test substance inhibits the heterodimer formation between the AIMP2DX2 protein and AIMP2 protein, wherein the test substance to inhibit the heterodimer formation between AIMP2DX2 protein and AIMP2 protein is evaluated as an anticancer agent.

The formation of the heterodimer between the AIMP2DX2 protein and AIMP2 protein is associated with cancer as demonstrated in the Examples described hereunder. Therefore, a substance capable of inhibiting the heterodimer formation is evaluated as a candidate for anticancer agent.
A method of screening for a formation of a heterodimer includes, for example a yeast-two-hybrid assay and an in vitro pull-down assay.
In a yeast two-hybrid assay firstly developed by Field & Song (1989), the AIMP2DX2 protein and AIMP2 protein can be used as either "bait" or "prey" (see, e.g., U.S. Patent 5, 283, 317; Zervos et al., Cell 72, 223-232(1993); Maduraetal., J. Biol. Chem. 268, 12046-12054(1993); Bartel et al., BioTechniques 14, 920-924(1993); Iwabuchi et al., Oncogene 8, 1693-1696(1993); and Brent W0 94/10300), to identify substances which inhibit the interaction of AIMP2DX2 with AIMP2 to form a heterodimer. The two-hybrid system is based on the modular nature of most transcription factors, which consist of separable DNA-binding and activation domains. Briefly, the assay utilizes two different DNA constructs. For example, in one construct, a polynucleotide encoding the AIMP2DX2 protein can be fused to a polynucleotide encoding the DNA binding domain of a known transcription factor (e.g. Lex). In the other construct a DNA sequence that encodes the AIMP2 protein can be fused to a polynucleotide that codes for the activation domain of the known transcription factor (e.g. B42).
If the test substance treated to cells expressing the two-hybrid system is able to inhibit the interaction between the AIMP2DX2 protein and AIMP2 protein, the DNA-binding and activation domains of the transcription factor are not brought into close proximity. This proximity allows transcription of a reporter gene (e.g. lac Z), which is operably linked to a transcriptional regulatory site responsive to the transcription factor. Expression of the reporter gene can be easily detected.
In an in vitro pull-down assay format, the AIMP2DX2 gene and AIMP2 gene can be used as either bait or prey. For example, in one construct for bait, the AIMP2 protein is fused to a protein that allows the AIMP2 protein to be bound to a solid support. For example, glutathione-S-transferase (GST) fusion proteins can be adsorbed onto glutathione Sepharose beads or glutathione derivatized microtiter plates. In the other construct for prey, the AIMP2DX2 protein is synthesized by an in vitro translation system (e.g., reticulocyte lysate) using ³⁵S. The radioactive AIMP2DX2 protein is added to the GST-AIMP2 protein bound to glutathione Sepharose beads together with the addition of the test substance. The reaction mixture is washed and the proteins bound to the Sepharose beads are eluted, followed by electrophoresis.
If the test substance added to the reaction mixture is able to inhibit the interaction between the AIMP2DX2 protein and AIMP2 protein, the electrophoresis result shows no band.

In a still further aspect of this invention, there is provided a method of screening for an anticancer agent which inhibits the expression of the AIMP2DX2 gene, comprising the steps of: (a) contacting a test substance to cells which express the AIMP2DX2 gene; and (b) determining whether the test substance inhibits the expression of the AIMP2DX2 gene or promotes the disruption of AIMP2DX2 protein, wherein the test substance to inhibit the expression of the AIMP2DX2 gene or promote the disruption of AIMP2DX2 protein is evaluated as an anticancer agent.
In the present method, the expression of the AIMP2DX2 gene is assessed at mRNA or protein level. Where the present method is carried out to detect the AIMP2DX2 mRNA expressed, it is preferably performed by RT-PCR using AIMP2DX2 specific primers described hereinabove. Where the present method is carried out to detect the AIMP2DX2 protein expressed, it is preferably performed by a variety of immunoassay processes such as Western blotting using AIMP2DX2 specific antibodies as described above.

The following specific examples are intended to be illustrative of the invention and should not be construed as limiting the scope of the invention as defined by appended claims.

### EXAMPLES

### METHODS

### Cell culture, chemicals and cell cycle measurement

Cells were maintained in RPMI-1640 containing 10% FBS. Mouse embryonic fibroblasts (MEFs) were isolated from 12.5-14.5 day embryos and cultivated in DMEM (Dulbecco's Modified Eagle Medium) containing 20% FBS. To evaluate the effect of TGF-β on cell cycle, cells were incubated with 2 ng/ml TGF-β in serum-free or 1% FBS-containing medium for 24 hr and harvested for FACS analysis. Cell proliferation was also determined by [³H] thymidine incorporation. Cells were incubated in serum-free medium with or without TGF-β for 20 hr, and then in the presence of 1 µCi/ml of [³H] thymidine for 4 hr. The incorporated thymidine was quantified by liquid scintillation counting as previously described (Kim, M. J. et al., Nat. Genet. 34, 330-336(2003)). TGF-β was purchased from R&D system, and anti-Smad2 and anti-Smad4 antibodies from Santa Cruz. The anti-AIMP2 antibody was prepared as follow: 500ug of the isolated AIMP2 protein mixed with 500 ul of complete adjuvant (Sigma, 1mg/ml) was injected subcutaneously into rabbit (male, New Zealand). After 7 days, 500 ug of the isolated AIMP2 protein mixed with 500 ul of complete adjuvant (1mg/ml) was injected into rabbit, and then the trials of injection were carried out 2 times at an interval of 7 days. After 3 days, anti-AIMP2 Ab was isolating from blood of rabbit.

### Immunoblotting and immunoprecipitation

Cells were treated with TGF-β for the indicated times and proteins were extracted with protease-containing RIPA buffer, separated by 10-12% SDS-PAGE, and immunoblotted with the specific antibodies using ECL system. For immunoprecipitation, the cell lysates were cleared by preincubation with IgG and agarose-conjugated protein A. After centrifugation, the supernatants were incubated with the specific antibody, and agarose-conjugated protein A for 2 hr. After washing with ice-cold PBS twice and RIPA once, the bound proteins were precipitated with the specific antibody, eluted and subjected to Western blot analysis.

### RT-PCR

The total RNAs were isolated following the protocol of the manufacturer (Qiagen). Briefly, the freshly prepared tissues (3 x 3 x 3mm) were chopped into small pieces, mixed with 350 µl lysis buffer and homogenized using homogenizer or syringe. After adding 350 µl of 70% ethanol, the lysates were inverted several times, loaded onto column, centrifuged at 13,000 RPM for 15 sec. After washing the column with wash buffer twice, RNAs were eluted with 40 µl RNase free DW. For reverse transcription, 1 µg of the isolated RNA was used as a template with the AIMP2specific primer (Fig. 8b). After the reaction, the mixture was diluted 3 fold with DW and 1 µl of its aliquot was used for 30 µl PCR reaction containing 0.5 µl dNTP (2.5 mM each), 0.5 µl of the indicated primers (each 10 pM), 1.5 µl DMSO and 0.1 µl Taq polymerase (5 U/µl).

### Suppression of AIMP2DX2 with si-RNA

To suppress the expression of AIMP2DX2, siRNAs against AIMP2DX2 were prepared. The sequence encoding siRNA against AIMP2DX2 was designed and cloned into *Sal*I and *Xba*I site of 16 IMG-700 vector system (Imgenex), followed by DNA sequencing for confirming the cloned sequence. H322 cells were transfected with 2 µg of si-DX2 expression vector. Following 3-hr incubation with DNA-liposome complex in 1 ml serum-free media, H322 cells were treated with 1 ml RPMI-1640 containing 20% FBS. Then, cells were incubated in serum-free medium with or without TGF-β for 20 hr.

### Yeast two-hybrid analysis

The cDNAs encoding human AIMP2 and Smad2 (and its deletion fragments) were obtained by PCR using specific primers. The PCR products of Smad2 and AIMP2 were digested with *Eco*RI and *Xho*I, and ligated into the same sites of pEG202 (LexA) and pJG4-5 (B42), respectively (Gyuris, J et al., a human G1 and S phase protein phosphatase that associates with Cdk2. Cell 75, 791-803(1993)). The interactions between Lex-Smad2 fragments and B42-AIMP2 were analyzed for their ability to grow on yeast medium containing X-gal as previously described (Rho, S. B. et al., Proc Natl Acad Sci U S A 96, 4488-93. (1999)).

### Construction of cells stably generating AIMP2DX2

The wild type MEFs were transfected with pcDNA-AIMP2DX2 or pcDNA itself and the transfectants were selected in DMEM medium containing 400 µg/ml G418. After removing the untransfected cells, the transfectants were cultured in the normal medium without G418 for 3 days, fixed with 2% PFA and stained with Giemsa.

### Immunostaining and histological analysis

The frozen tissue slides were fixed with 2% paraformaldehyde and washed with ice cold PBS. After blocking and permeablization with PBS containing 0.2% Triton X-100 (PBST) and 1% BSA, the slides were incubated with anti-AIMP2 antibody for 2 hr. After washing with PBS, they were also incubated with anti-rabbit goat IgG-FITC and propidium iodide (50 µg/ml) for 1 hr, washed with PBS, mounted, and observed under a confocal microscopy.

### Construction of AIMP2DX2 expression vector

To construct the AIMP2DX2 expressing vector, cDNA of AIMP2DX2 was cloned into pcDNA3.1-myc. First, AIMP2DX2 from H322 cDNA was amplified using primers with linker for EcoRI and *Xho*I and cloned into pcDNA3.1-myc (Invitrogen) using *Eco*RI and *Xho*I. The pcDNA3.1-myc/AIMP2DX2 vector to express AIMP2DX (Fig. 12) was introduced into *E. coli* DH5α, which was deposited on October 25, 2004 in the International Depository Authority, the Korean Collection for Type Cultures (KCTC) and was given accession number KCTC 10710BP.

### Generation and Characterization of Knock-out mouse

The mutation on the genomic DNA of a mouse was generated by gene trap method (Zambrowicz et al, Nature, 9, 608-611, 1998). The gene trap vector was used to generate the random mutant library (OmniBank library, Lexicon Genetics, USA) of the embryonic stem cells derived from 129/SvEvBrd mouse. In the library, OST7994 clone in which the AIMP2DX2 gene was disrupted were identified. Using the above clone, the C57/BL6 heterozygous mice were generated by following the standard protocol of Lexicon Genetics. The heterozygous mice were then crossed to obtain homozygous mutant mice. The isolated 13.5 day mouse embryos were minced with razor blade, trypsinized and plated in petri dish for cultivation.
The mutation was confirmed by Southern blot and PCR analyses using the genomic DNAs isolated from mouse embryonic fibroblast (MEF) cells. The AIMP2 expression was determined by Northern and Western blot analyses. The location of the gene trap insertion was determined by sequencing of genomic DNA. For Southern blotting, the genomic DNA was isolated from the mouse tail and digested with SacI. The cleaved DNA fragments were separated by denaturing gel electrophoresis and were hybridized with the radioactively labeled 1.2 kb PCR product of the AIMP2 gene with primers, SEQ NOs 17 and 18. The hybridized bands were detected using phosphorimage analyzer. The isolated genomic DNA was also used for PCR analysis with the specific pairs of the primers, SEQ NOs 19 and 20, SEQ NOs 20 and 21.
For Northern analysis, total cellular RNA was prepared from mouse embryonic fibroblast cells using RNeasy Midi kit (QIAGEN) according to the manufacturer's instructions. The isolated RNAs (30ug) were separated by electrophoresis in 1.2% agarose/2.2M formaldehyde gels, transferred to Hybond N membranes (Amersham) and then fixed to the membrane by UV irradiation. The RNAs on the membrane were hybridized with the specific probes generated by PCR of the AIMP2 cDNA using the ExpressHyb hybridization solution (CLONTECH). The proteins were extracted from mouse embryonic fibroblast (MEF) cells, separated by SDS-PAGE and subjected to Western blotting with anti-AIMP2 polyclonal antibody as described (Park et al, 274, 16673-16676, 1999; Kim et al, J Biol Chem, 275, 21768-21772, 2000).

### Lung cancer formation

32 AIMP2^{+/-} mice (19 male and 13 female mice) and 25 AIMP2^{+/+} mice (14 male and 11 female mice) were used for experiment. We induced lung tumor through the intraperitoneal injection of chemical carcinogen, benzo-(α)-pyrene (BP, 100 mg/kg) into AIMP2^{+/+} and AIMP2^{+/-} mice, and monitored the tumor formation in lung. As a control group, 3 AIMP2^{+/+} (2 male and 1 female) and 5 AIMP2^{+/-} mice (4 male and 1 female) were injected with bical solution (10 % DMSO and 35% PEG 40 in saline).

### Skin cancer model using knock-out mouse

33 of wild-type (WT) and AIMP2 knock-out (KO) mice of both sexes were divided into following groups : WT male control (3), WT male treated (6), WT female control (2), WT female treated (6), KO male control (2), KO male treated (6), KO female control (3), and KO female treated groups (5).
A single dose (0.2 µmol/0.2 ml acetone) of 7,12-dimethylbenz[a]anthracene (DMBA) was topically applied to shaven backs of mice belonging to treated groups, while the mice of control groups were treated with acetone only. After one week, DMBA-initiated mice were treated with 12-O-tetradecanoyl-phorbol-13-acetate (TPA, 10 nmol/0.2 ml acetone) twice a week for 21 weeks. Control animals were treated with acetone only throughout the study. Starting from 1 week of promoter treatment, tumors of at least 1 mm diameter were counted every week. After 21 weeks mice were sacrificed and tumor samples corrected for subsequent biochemical analysis.

### EXAMPLE 1

### Functional importance and working mode of AIMP2 in TGF-β signaling

To see the importance of AIMP2 in TGF-β signaling, we compared the responses of the AIMP2^{+/+} and AIMP2^{/-} MEFs to TGF-β-induced growth arrest, nuclear translocation of Smad2/3 and interaction of AIMP2 with Smad2/3. While the growth of the wild type cells was suppressed by TGF-β, the AIMP2deficient cells did not respond to the signal as determined by thymidine incorporation, colony formation and flow cytometry (Fig. 1a, 1b and 1c, respectively). When MEFs were treated with TGF-β, Smad2 and Smad3 were translocated to nuclei in the normal cells, but not in the AIMP2^{/-} cells (Fig. 1d). All of these results suggest the functional importance of AIMP2 in TGF-β signaling via Smad2 and Smad3.
The possible interaction of AIMP2 with Smad2 and 3 was checked by coimmunoprecipitation. AIMP2 showed the interaction with Smad2/3 that was enhanced by TGF-β (Fig. 1e). The direct interaction of AIMP2 with two R-Smads was also confirmed by yeast two hybrid and in vitro pull-down assays (Figs. 2 and 7). The amount of AIMP2 bound to Smad2/3 was increased according to the induction of AIMP2 by TGF β (Fig. 2a) when the AIMP2 level was increased by transfection, the expression of the TGF-β target genes was enhanced, suggesting its stimulatory role in TGF-β signaling (Fig. 2b). Since AIMP2 binds to both of Smad2 and 3, we expect that it would work to these two R-Smads in a similar way and thus focused on its relationship to Smad2 in more detail. The TGF-β-dependent phosphorylation of Smad2 was suppressed in the AIMP2deficient MEFs, but restored when AIMP2 was introduced to AIMP2^{/-} cells (Fig. 2c). The domain of Smad2 involved in the interaction with AIMP2 was determined by yeast two hybrid (Fig. 2d) and in vitro pull-down assay (Fig. 7). The two experiments revealed that the interaction involves the MH2 domain of Smad2.
To determine the working mode of AIMP2 in TGF-β signaling, the TGF-β-induced association of Smad2 with TGF-β receptor was compared in the normal and AIMP2 deficient cells. The cells were treated with TGF-β and the association of Smad2 and the receptor was monitored by coimmunoprecipitation of type I receptor with Smad2 at time interval. While the TGF-β-induced Smad2 binding to TGF-β receptor was observed at early time point and decreased in the wild type cells, the receptor bound to Smad2 was accumulated in the late stage after TGF-β treatment in the AIMP2deficient cells (Fig. 2e). In the TGF-β-induced phosphorylation of Smad2, the phosphorylated Smad2 was gradually increased in the wild type cells, but severely suppressed in the AIMP2^{-/-} cells (Fig. 2f). These results demonstrate that AIMP2 plays a critical role in the TGF-β induced phosphorylation of R-Smads via its direct interaction with R-Smad2.

### EXAMPLE 2

### Suppression of AIMP2 and generation of its deletion variant in cancer cells

To explore the possible association of AIMP2 with cancer formation, the variation of AIMP2 level in different cancer cell lines was observed[A549 (lung epithelial carcinoma), DU145 (prostate carcinoma), HCT116 (human colorectal carcinoma), H460 (large cell lung carcinoma), H322 (lung bronchioalveolar carcinoma) and H290 (mesothelioma cancer cell line) available from ATCC]. Three of the six tested cell lines showed lower AIMP2 level in Western blot (Fig. 3a) and FACS analyses (Fig. 3b). All of the cell lines with low AIMP2 level expressed the normal level of the TGF-β type II receptor and retained its kinase activity, implying that the low AIMP2 level does not result from the mal-functionality of the receptor. To determine whether the variation of AIMP2 level resulted from the difference in transcription, RT-PCR with different combinations of the AIMP2 specific primers was performed. The AIMP2 gene consists of four exons (Fig. 8a). When the primers of SEQ ID Nos. 3 and 4 were used to generate AIMP2 cDNA spanning exon 3 and 4, the decrease of AIMP2 transcript was not observed in the cells showing the reduced level of AMP2 (Fig. 3c, first row), suggesting that it does not result from lower transcription. When the primers of SEQ ID Nos. 5 and 6 generating the transcript from exon 1 to 3 were used, not only the transcript of the expected size, but also a smaller one (Fig. 3c, second row) were obtained. Sequencing analysis of this small transcript revealed that it lacks exon 2 encoding 69 aa of AIMP2 (Fig. 8b). To confirm the generation of this smaller transcript, the primer of SEQ ID No.8 (Fig. 8b, primer DX-B) targeting to the junction sequence of exon 1 and 3 that is generated by the deletion of exon 2 was designed, and RT-PCR was conducted with the primer of SEQ ID No.7. The cell lines expressing lower AIMP2 level generated the smaller transcript (designated DX2, Fig. 3c, second and third rows).
Western blot analysis with anti-AIMP2 antibody detected only the full-length AIMP2, but not DX2 (Fig. 2a), implying that DX2 may be very unstable at protein level. Immunofluorescence staining also demonstrated the lower AIMP2 level in H460, H322 and H290 (Fig. 3d). To exclude the possibility of staining artifact, H460 and DU145 cells were co-cultivated in the same plate and stained AIMP2. Again, the staining intensity of AIMP2 in H460 was much weaker than that in DU145 (Fig. 9a). In addition, the TGF-β-dependent nuclear localization of AIMP2 was not observed in the cells with low AIMP2 expression (Fig. 3d, bottom row). To address the linkage between the AIMP2 level and functionality of TGF β, we measured growth suppression by TGF-β in these cell lines. While the growth of A549 and DU145 cells was suppressed by TGF-β, the cells with low AIMP2 level did not respond to TGF-β (Fig. 3e). This result is consistent with previous reports that A549 is sensitive whereas H460 is resistant to TGF-β signal (Osada, H. et al. Cancer Res. 61, 8331-8339(2001); Kim, T. K. et al., Lung Cancer 31, 181-191(2001)). In addition, the target genes were induced by TGF-β in A549 and DU145, but not in H322 and two other cell lines with low AIMP2 (Fig. 3f).

### EXAMPLE 3

### The inactive deletion variant forms a complex with functional AIMP2

To comprehend the causal relationship of the DX2 generation and suppression of AIMP2, the change of the AIMP2 level was checked after transfection of DX2 into DU145. AIMP2 was reduced in the DX2-transfected cells, as demonstrated by Western blot (Fig. 4a, first row) and FACS analyses (Fig. 9b). Moreover, the expression of the AIMP2 target, c-myc, was elevated by the introduction of DX2 (Fig. 4a, third row). DX2 also relieved the growth arrest by TGF-β (Figs. 4b and 9c). We then investigated how DX2 would affect the functional AIMP2 (AIMP2F). Since AIMP2 has a potential to form a homodimer (Quevillon, S. et al., J. Mol. Biol. 285, 183-195(1999); and Kim, J. Y. et al., Proc. Natl. Acad. Sci. USA 99, 7912-7916(2002)), we examined whether AIMP2 DX2 would interact with AIMP2F by yeast two hybrid assay. AIMP2DX2 showed the interaction with AIMP2F, but not with itself (Fig. 4c). In *in vitro* pull-down assay, both of radioactively synthesized AIMP2F and -DX2, but not DX2, were co-purified with GST-AIMP2F (Fig. 4d), proving the direct interaction between AIMP2F and -DX2. To see whether DX2 is active in TGF-β signaling, we tested its interaction with Smad2 by yeast two hybrid assay. While AIMP2 interacted with Smad2 as well as FBP that is the known target of AIMP2 (Kim, M. J. et al., Nat. Genet. 34, 330-336(2003)), DX2 did not bind to any of these proteins, suggesting that it would be functionally inactive (Fig. 4e).
To understand how the heterodimer formation would suppress AIMP2, we checked whether the AIMP2 level is controlled by proteasome-dependent degradation process. The DX2-producing H322 cells were treated with the proteasome inhibitor, ALLN (Zhou, M., et al., J. Biol. Chem. 271, 29769-24775(1996)) and tested whether the AIMP2 level is increased by the blockage of proteasome. The AIMP2 level was significantly increased by the treatment of ALLN as shown by Western blotting (Fig. 4f), immunofluorescence staining (Fig. 4g) and flow cytometry (Fig. 9d), suggesting that its cellular level would be controlled by proteasome-mediated degradation. In addition, AIMP2DX2 form was also detected by the inhibition of proteasome (Fig. 4f), confirming the notion that DX2 would be unstable due to the rapid proteasome-dependent degradation. The low intensity of AIMP2DX2 appears to result from its lower transcription compared to the normal AIMP2, as demonstrated by RT-PCR analysis (Fig. 3c) and/or less efficient recognition by anti-AIMP2 antibody. Since AIMP2 degradation is mediated by proteasome, we tested whether its ubiquitination is promoted by DX2. When AIMP2 was immunoprecipitated from the control and DX2-transfected cells that were treated with ALLN and blotted with anti-ubiquitin antibody, higher amount of the ubiquitinated AIMP2 was observed in the DX2-transfected cells compared to that in control cells (Fig. 4h). Combined together, DX2 appears to work as a dominant negative mutant to form an inactive complex with AIMP2 that is rapidly driven to degradation process.

### EXAMPLE 4

### AIMP2DX2 inactivates TGF-β signaling and promotes cell growth

DX2 was transfected into MEFs and monitored its effect on cell growth by microscopic analysis and colony formation. The cell growth was significantly enhanced by the introduction of DX2 (Fig. 5a). We then introduced siRNA (si-DX2) that specifically suppresses the DX2 transcript and checked whether it can restore the normal level of AIMP2 and TGF-β signaling in H322 cells expressing DX2. When H322 cell was transfected with the vector expressing siRNA of No.4 or No 5, elevation of cell death (left) and generation of cell-cycle arrest (right) by the TGF-β were measured by FACS (Fig. 5d). To test whether normal level of AIMP2 and TGF-β signaling can be repaired by the siRNA, the vector generating siRNA(si-DX2) of No. 4 specifically suppressing DX2-specific expression, were transfected to the H322 cell expressing DX2. si-DX2 ablated the DX2 transcript (Fig. 5e top) and resumed the normal AIMP2 level and TGF-β-induced phosphorylation of Smad2 (Fig. 5e bottom and Fig. 5f), nuclear localization of p-Smad2 (Fig. 5f), TGF-β-dependent reporter expression (Fig. 5g) and growth arrest (Fig. 5h). All of these results demonstrated the disruptive effect of DX2 on TGF-β signaling and cell growth control.

### EXMAPLE 5

### Association of AIMP2 deletion variant with human lung cancer

Since the reduction of AIMP2 is frequently detected in different cancer cell lines (Figs. 3a, 3b and 3d), and loss of AIMP2 leads to hyper-proliferation of lung cells (Kim, M. J. et al., Nat. Genet. 34, 330-336(2003)), we examined the association of AIMP2 abnormality with lung cancer formation. We induced lung tumor through the intraperitoneal injection of chemical carcinogen, benzo-(α)-pyrene (BP, Wang, Y. et al., Cancer Res. 63, 4389-4395 (2003)) into AIMP2^{+/+} and AIMP2^{+/-} mice, and monitored the tumor formation in lung. From 6 weeks after the administration of BP, lung tumors were observed at 50-70% frequency in AIMP2^{+/-} mice, and at about 30% in the wild type littermates (Fig. 6a), implying that the heterozygous mice are more susceptible to BP-induced tumorgenesis. Three out of four lungs isolated from AIMP2^{+/-} mice showed tumors, while only one developed tumors among three AIMP2^{+/+} mice and all of these tumors generated DX2 (Fig. 10), further supporting the relevance of DX2 to tumor formation. We then compared the AIMP2 level in the inflammatory but non-tumor, and tumor tissues in human lung. DX2 was generated only in tumor tissues (Fig. 6b). To exclude the possibility that the AIMP2 level and DX2 formation may vary depending on different individuals, we carried out RT-PCR and immunofluorescence analyses in the normal and tumor pairs isolated from the same patients. DX2 was not detected in one case showing the normal AIMP2 level in cancer region (Figs. 6e and 6f, patient 22872).

### EXMAPLE 6

### Association of AIMP2, AIMP2DX2 and its deletion variant

The relationship of AIMP2, AIMP2DX2 and its deletion variant with cancer was examined in human tissues and cancer cell lines.
AIMP2DX2 formation in normal and cancer tissue obtained from hepatocellular carcinoma was evaluated by RT-PCR. Also, the formation of AIMP2DX2 in different cancer cell lines was examined (Fig. 11a) The cells were seeded in 6-well plate at a density of 1x10⁵ and harvested 24 hours later. The harvested cells were lysated using GSS buffer, mixed with 4% isoamylalcohol containing acidic phenol (pH 4.3) and chloroform and then centrifuged. The supernatant obtained by centrifugation were precipitated with isopropanol and centrifuged again, and then the pellet was obtained subsequently. The pellet was washed with ethanol and dissolved in DW. RNA was reverse transcribed an 42 °C for 1 hr using M-MLV reverse transcriptase (invitrogen) and then PCR was performed 38 cycles of 95°C for 1 min, 60°C for 1 min, and 72°C for 1 min using primers SEQ Nos. 16 and 17. AIMP2DX2 were expressed in lung cancer cell lines (H226, H460, H322 and H290), embryo kidney cell line (293), osteosarcoma cell line (SaOS2), skin cancer cell line (HaCAT), and breat cancer cell line (MCF7) (Fig. 11b).

### EXAMPLE 7

### Study of tumorgenesis using AIMP2 knock-out mouse

Susceptibility to tumor was observed in AIMP2DX2 knock-out mice and wild mice.
As a result, the number of mouse having tumors on the skin and the tumor number of mouse were increased significantly in AIMP2DX2 knock-out mouse. In detail, when mice's tumorgenesis was induced with DMBA and promoted with TPA, the rate of tumor formation in AIMP2 knock-out female mice were increased about 1.3 folds than in WT female control mice. Also, the rate of tumor formation in AIMP2 knock-out male mice were increased about 2 folds than in WT male control mice (Fig 12b). The number of tumor to be formed in AIMP2 knock-out female mice were increased about 4 folds than in WT female control mice. The number of tumor to be formed in AIMP2 knock-out male mice were increased about 2 folds than in WT male control mice (Fig. 12c and Fig. 12d).
## Claims

1. A AIMP2DX2 protein comprising
(a) a AIMP2 amino acid sequence in which the exon 2 region of the AIMP2 amino acid sequence is deleted, wherein AIMP2 has the sequence: or
(b) the amino acid sequence of SEQ ID NOs: 2.

2. A nucleic acid molecule comprising a nucleotide sequence encoding the AINP2DX2 protein of claim 1.

3. The nucleic acid molecule according to claim 2, wherein said nucleic acid molecule comprises the nucleotide sequence of SEQ ID NO:1.

4. A recombinant vector, which comprises a nucleic acid molecule as claimed in claim 2 or claim 3.

5. A host cell which is transformed with the recombinant vector of claim 4, wherein the cell is a non-human cell or is an isolated human cell that is not a human embryonic stem cell.

6. A method for producing the AIMP2DX2 protein of claim 1, comprising culturing the cell of claim 5.

7. An antibody specific to the ATMP2DX2 protein of claim 1.

8. A diagnostic kit for cancer, which comprises an antibody as claimed in claim 7.

9. A method for screening for AIMP2DX2 protein as a cancer marker, which comprises the steps of:
(a) providing a sample to be assayed; and
(b) detecting in said sample expression of a nucleic acid molecule as claimed in claim 2 or claim 3, wherein detection of the expression of the nucleic acid molecule is indicative of cancer.

10. The method according to claim 9, wherein said detecting in step (b) is carried out by contacting said sample with an antibody as claimed in claim 7.

11. The method according to claim 9, wherein said detecting in step (b) is carried out by an amplification reaction or a hybridization reaction.

12. The method according to claim 11, wherein said amplification reaction is carried out by RT-PCR (reverse transcription-polymerase chain reaction) using a primer capable of differentiating an mRNA of AIMP2DX2 of claim 1 from an mRNA of AIMP2 or said hybridization reaction is carried out by using a probe capable of differentiating an mRNA of AIMP2DX2 of claim 1 from an mRNA of AIMP2, wherein AIMP2 has the sequence:

13. The method according to claim 12, wherein the sequence of said primer comprises SEQ ID Nos.5 and 6, 7 and 8 or 16 and 6; or the sequence of said probe comprises SEQ ID No. 8 or 16.

14. A nucleic acid molecule comprising the nucleotide sequence of SEQ ID No.3, 4, 5, 6, 7, 8 or 16.

15. A siRNA nucleic acid molecule specific to mRNA of the AIMP2DX2 protein of claim 1, wherein said siRNA nucleic acid molecule comprises sense and antisense RNA encoding for the nucleotide sequence of SEQ ID Nos. 9 and 10, SEQ ID Nos. 11 and 12 or SEQ ID Nos. 13 and 14.

16. An antisense oligonucleotide which is complementary to a junction region between exons 1 and 3 of an mRNA of the AIMP2DX2 protein of claim 1.

17. A pharmaceutical composition for treating cancer comprising the siRNAs nucleic acid molecule of claim 15, or the antisense oligonucleotide of claim 16.

18. The siRNAs nucleic acid molecule of claim 15, or the antisense oligonucleotide of claim 16 for use in treating cancer.

19. The use of the siRNA nucleic acid molecule of claim 15, or the antisense oligonucleotide of claim 16 in the manufacture of a medicament for treating cancer.

20. A method of screening for an anticancer agent which inhibits the formation of a heterodimer between the AIMP2DX2 protein of claim 1 and the AIMP2 protein, comprising the steps of:
(a) contacting a test substance to a composition which comprises the AIMP2DX2 protein of claim 1 and the AIMP2 protein; and
(b) determining whether the test substance inhibits the heterodimer formation between the AIMP2DX2 protein of claim 1 and the AIMP2 protein,
wherein the test substance to inhibit the heterodimer formation between the AIMP2DX2 protein of claim 1 and the AIMP2 protein is evaluated as an anticancer agent, and wherein AIMP2 has the sequence:

21. The method according to claim 20, wherein the method is performed by a yeast-two-hybrid assay or in vitro pull-down assay.

22. A method of screening for an anticancer agent which inhibits the expression of the gene encoding the AIMP2DX2 protein of claim 1, or promotes the disruption of the AIMP2DX2 protein of claim 1, comprising the steps of:
(a) contacting a test substance to cells which express the AIMP2DX2 gene; and
(b) determining whether the test substance inhibits the expression of the AIMP2DX2 gene or promotes the disruption of the AIMP2DX2 protein,
wherein the test substance to inhibit the expression of the AIMP2DX2 gene is evaluated as an anticancer agent.

23. The method according to claim 22, wherein the method is performed by RT-PCR or Western blot.

## Patentansprüche

1. AIMP2DX2-Protein umfassend
(a) eine AIMP2-Aminosäuresequenz, in der die Exon 2-Region der AIMP2-Aminosäuresequenz deletiert ist, wobei AIMP2 die folgende Sequenz aufweist:
(b) die Aminosäuresequenz nach SEQ ID NR: 2.

2. Nukleinsäuremolekül umfassend eine Nukleotidsequenz, die für das AIMP2DX2-Protein nach Anspruch 1 kodiert.

3. Nukleinsäuremolekül nach Anspruch 2, wobei das Nukleinsäuremolekül die Nukleotidsequenz nach SEQ ID NR: 1 umfasst.

4. Rekombinanter Vektor, der ein Nukleinsäuremolekül nach Anspruch 2 oder 3 umfasst.

5. Wirtszelle, die mit dem rekombinanten Vektor nach Anspruch 4 transformiert ist, wobei es sich bei der Zelle um eine nicht humane Zelle oder um eine isolierte humane Zelle handelt, die keine humane embryonale Stammzelle ist.

6. Verfahren zur Herstellung des AIMP2DX2-Proteins nach Anspruch 1, umfassend das Kultivieren der Zelle nach Anspruch 5.

7. Antikörper, der für das AIMP2DX2-Protein nach Anspruch 1 spezifisch ist.

8. Kit zur Krebsdiagnose, das einen Antikörper nach Anspruch 7 umfasst.

9. Verfahren zum Screening für das AIMP2DX2-Protein als Krebsmarker, umfassend die folgenden Schritte:
(a) Bereitstellen einer zu untersuchenden Probe; und
(b) Detektieren der Expression eines Nukleinsäuremoleküls nach Anspruch 2 oder 3 in dieser Probe, wobei die Detektion der Expression des Nukleinsäuremoleküls indikativ für Krebs ist.

10. Verfahren nach Anspruch 9, wobei die Detektion in Schritt (b) erfolgt, indem die Probe mit einem Antikörper nach Anspruch 7 in Kontakt gebracht wird.

11. Verfahren nach Anspruch 9, wobei die Detektion in Schritt (b) mittels einer Amplifikations- oder Hybridisierungsreaktion erfolgt.

12. Verfahren nach Anspruch 11, wobei die Amplifikationsreaktion mittels einer RT-PCR (Reverse Transkriptase-Polymerase-Kettenreaktion) unter Verwendung eines Primers erfolgt, der dazu in der Lage ist, zwischen einer mRNA von AIMP2DX2 nach Anspruch 1 und einer mRNA von AIMP2 zu differenzieren, oder die Hybridisierungsreaktion unter Verwendung einer Sonde erfolgt, die dazu in der Lage ist, zwischen einer mRNA von AIMP2DX2 nach Anspruch 1 und einer mRNA von AIMP2 zu differenzieren, wobei AIMP2 die folgende Sequenz aufweist:

13. Verfahren nach Anspruch 12, wobei die Sequenz des Primers SEQ ID NRn: 5 und 6, 7 und 8 oder 16 und 6 umfasst oder die Sequenz der Sonde SEQ ID NR: 8 oder 16 umfasst.

14. Nukleinsäuremolekül umfassend die Nukleotidsequenz nach SEQ ID NR: 3, 4, 5, 6, 7, 8 oder 16.

15. siRNA-Nukleinsäuremolekül, das für mRNA des AIMP2DX2-Proteins nach Anspruch 1 spezifisch ist, wobei das siRNA-Nukleinsäuremolekül Sense- und Antisense-RNA umfasst, die für die Nukleotidsequenz von SEQ ID NRn: 9 und 10, SEQ ID NRn: 11 und 12 oder SEQ ID NRn: 13 und 14 kodiert.

16. Antisense-Oligonukleotid, das zu einer Verbindungsregion zwischen den Exons 1 und 3 einer mRNA des AIMP2DX2-Proteins nach Anspruch 1 komplementär ist.

17. Pharmazeutische Zusammensetzung zur Behandlung von Krebs, umfassend das siRNA-Nukleinsäuremolekül nach Anspruch 15 oder das Antisense-Oligonukleotid nach Anspruch 16.

18. siRNA-Nukleinsäuremolekül nach Anspruch 15 oder Antisense-Oligonukleotid nach Anspruch 16 zur Verwendung bei der Behandlung von Krebs.

19. Verwendung des siRNA-Nukleinsäuremoleküls nach Anspruch 15 oder des Antisense-Oligonukleotids nach Anspruch 16 bei der Herstellung eines Medikaments zur Behandlung von Krebs.

20. Verfahren zum Screening für einen Antikrebswirkstoff, der die Bildung eines Heterodimers zwischen dem AIMP2DX2-Protein nach Anspruch 1 und dem AIMP2-Protein inhibiert, umfassend die folgenden Schritte:
(a) Inkontaktbringen einer Testsubstanz mit einer Zusammensetzung, die das AIMP2DX2-Protein nach Anspruch 1 und das AIMP2-Protein umfasst; und
(b) Bestimmen, ob die Testsubstanz die Heterodimerbildung zwischen dem AIMP2DX2-Protein nach Anspruch 1 und dem AIMP2-Protein inhibiert,
wobei die Testsubstanz zur Inhibition der Heterodimerbildung zwischen dem AIMP2DX2-Protein nach Anspruch 1 und dem AIMP2-Protein als Antikrebswirkstoff evaluiert wird und wobei AIMP2 die folgende Sequenz aufweist:

21. Verfahren nach Anspruch 20, wobei das Verfahren mittels eines Hefe-Zwei-Hybrid-Assays oder eines *in vitro* Pull-Down-Assays durchgeführt wird.

22. Verfahren zum Screening für einen Antikrebswirkstoff, der die Expression des für das AIMP2DX2-Protein nach Anspruch 1 kodierenden Gens inhibiert oder die Disruption des AIMP2DX2-Proteins nach Anspruch 1 fördert, umfassend die folgenden Schritte:
(a) Inkontaktbringen einer Testsubstanz mit Zellen, die das AIMP2DX2-Gen exprimieren; und
(b) Bestimmen, ob die Testsubstanz die Expression des AIMP2DX2-Gens inhibiert oder die Disruption des AIMP2DX2-Proteins fördert,
wobei die Testsubstanz zur Inhibition der Expression des AIMP2DX2-Gens als Antikrebswirkstoff evaluiert wird.

23. Verfahren nach Anspruch 22, wobei das Verfahren mittels RT-PCR oder Western-Blot durchgeführt wird.

## Revendications

1. Protéine AIMP2DX2 comprenant
(a) une séquence d'acide aminé AIMP2 dans laquelle la région de l'exon 2 de la séquence d'acides aminés AIMP2 est supprimée, où AIMP2 a la séquence suivante : ou
(b) la séquence d'acide aminé de SEQ ID N° 2.

2. Molécule d'acide nucléique comprenant une séquence de nucléotides codant pour la protéine AIMP2DX2 de la revendication 1.

3. Molécule d'acide nucléique selon la revendication 2, ladite molécule d'acide nucléique comprenant la séquence de nucléotides de SEQ ID N° 1.

4. Vecteur recombinant, qui comprend une molécule d'acide nucléique selon la revendication 2 ou la revendication 3.

5. Cellule hôte qui est transformée avec le vecteur recombinant de la revendication 4, la cellule étant une cellule non humaine ou une cellule humaine isolée qui n'est pas une cellule souche d'embryon humain.

6. Procédé de production de la protéine AIMP2DX2 de la revendication 1, comprenant la mise en culture de la cellule de la revendication 5.

7. Anticorps spécifique de la protéine AIMP2DX2 de la revendication 1.

8. Nécessaire de diagnostic pour le cancer, qui comprend un anticorps selon la revendication 7.

9. Procédé de dépistage de la protéine AIMP2DX2 comme marqueur du cancer, qui comprend les étapes consistant à :
(a) fournir un échantillon à doser ; et
(b) détecter dans ledit échantillon l'expression d'une molécule d'acide nucléique selon la revendication 2 ou la revendication 3, la détection de l'expression de la molécule d'acide nucléique étant indicative du cancer.

10. Procédé selon la revendication 9, dans lequel ladite détection à l'étape (b) est réalisée par mise en contact dudit échantillon avec un anticorps selon la revendication 7.

11. Procédé selon la revendication 9, dans lequel ladite détection à l'étape (b) est réalisée par une réaction d'amplification ou une réaction d'hybridation.

12. Procédé selon la revendication 11, dans lequel ladite réaction d'amplification est réalisée par RT-PCR (réaction en chaîne par polymérase après transcription inverse) en utilisant une amorce capable de différencier un ARNm de AIMP2DX2 de la revendication 1 d'un ARNm de AIMP2, ou ladite réaction d'hybridation est réalisée par l'utilisation d'une sonde capable de différencier un ARNm de AIMP2DX2 de la revendication 1 d'un ARNm de AIMP2, AIMP2 ayant la séquence:

13. Procédé selon la revendication 12, dans lequel la séquence de ladite amorce comprend SEQ ID N° 5 et 6, 7 et 8 ou 16 et 6 ; ou la séquence de ladite sonde comprend SEQ ID N° 8 ou 16.

14. Molécule d'acide nucléique comprenant la séquence de nucléotides de SEQ ID N° 3, 4, 5, 6, 7, 8 ou 16.

15. Molécule d'acide nucléique de ARNsi spécifique de l'ARNm de la protéine AIMP2DX2 de la revendication 1, ladite molécule d'acide nucléique d'ARNsi comprenant un ARN sens et anti-sens codant pour la séquence de nucléotides des SEQ ID N° 9 et 10, SEQ ID N° 11 et 12 ou SEQ ID N° 13 et 14.

16. Oligonucléotide anti-sens qui est complémentaire d'une région de jonction entre les exons 1 et 3 d'un ARNm de la protéine AIMP2DX2 de la revendication 1.

17. Composition pharmaceutique destinée au traitement du cancer comprenant la molécule d'acide nucléique d'ARNsi de la revendication 15, ou l'oligonucléotide anti-sens de la revendication 16.

18. Molécule d'acide nucléique d'ARNsi de la revendication 15, ou oligonucléotide anti-sens de la revendication 16 à utiliser dans le traitement du cancer.

19. Utilisation d'une molécule d'acide nucléique d'ARNsi de la revendication 15, ou de l'oligonucléotide anti-sens de la revendication 16 dans la fabrication d'un médicament pour le traitement du cancer.

20. Procédé de dépistage d'un agent anti-cancéreux qui inhibe la formation d'un hétérodimère entre la protéine AIMP2DX2 de la revendication 1 et la protéine AIMP2, comprenant les étapes consistant à :
(a) mettre en contact une substance d'essai avec une composition qui comprend la protéine AIMP2DX2 de la revendication 1 et la protéine AIMP2 ; et
(b) déterminer si la substance d'essai inhibe la formation d'hétérodimère entre la protéine AIMP2DX2 de la revendication 1 et la protéine AIMP2,
dans lequel la substance d'essai qui doit inhiber la formation d'hétérodimère entre la protéine AIMP2DX2 de la revendication 1 et la protéine AIMP2 est évaluée en tant qu'agent anti-cancéreux, et dans lequel AIMP2 a la séquence suivante :

21. Procédé selon la revendication 20, dans lequel le procédé est réalisé par un dosage double hybride en levure ou un dosage pull-down in vitro.

22. Procédé de dépistage pour un agent anti-cancéreux qui inhibe l'expression du gène codant pour la protéine AIMP2DX2 de la revendication 1, ou favorise la dissociation de la protéine AIMP2DX2 de la revendication 1, comprenant les étapes consistant à :
(a) mettre en contact une substance d'essai avec des cellules qui expriment le gène AIMP2DX2 ; et
(b) déterminer si la substance d'essai inhibe l'expression du gène AIMP2DX2 ou favorise la dissociation de la protéine AIMP2DX2,
dans lequel la substance d'essai qui doit inhiber l'expression du gène AIMP2DX2 est évaluée en tant qu'agent anti-cancéreux.

23. Procédé selon la revendication 22, dans lequel le procédé est réalisé par RT-PCR ou buvardage de Western.


<110> Seoul National University Industry Foundation
<120> Use of AIMP2DX2 for the diagnosis and treatment of cancer
<130> P42707EP-K
<140> EP 05819055.4
   <141> 2005-11-18
<150> KR20040097164
   <151> 2004-11-24
<150> KR20050039073
   <151> 2005-05-10
<160> 21
<170> KopatentIn 1.71
<210> 1
   <211> 756
   <212> DNA
   <213> Homo sapiens
<220>
   <221> CDS
   <222> (1)..(753)
   <223> p38DX2 amino acid sequence
<400> 1
   atg ccg atg tac cag gta aag ccc tat cac ggg ggc ggc gcg cct ctc        48
   Met Pro Met Tyr Gln Val Lys Pro Tyr His Gly Gly Gly Ala Pro Leu
    1               5                   10                  15
   cgt gtg gag ctt ccc acc tgc atg tac cgg ctc ccc aac gtg cac ggc        96
   Arg Val Glu Leu Pro Thr Cys Met Tyr Arg Leu Pro Asn Val His Gly
               20                  25                  30
   agg agc tac ggc cca gcg ccg ggc gct ggc cac gtg cag gat tac ggg       144
   Arg Ser Tyr Gly Pro Ala Pro Gly Ala Gly His Val Gln Asp Tyr Gly
           35                  40                  45
   gcg ctg aaa gac atc gtg atc aac gca aac ccg gcc tcc cct ccc ctc       192
   Ala Leu Lys Asp Ile Val Ile Asn Ala Asn Pro Ala Ser Pro Pro Leu
       50                  55                  60
   tcc ctg ctt gtg ctg cac agg ctg ctc tgt gag cac ttc agg gtc ctg       240
   Ser Leu Leu Val Leu His Arg Leu Leu Cys Glu His Phe Arg Val Leu
   65                  70                  75                  80
   tcc acg gtg cac acg cac tcc tcg gtc aag agc gtg cct gaa aac ctt       288
   Ser Thr Val His Thr His Ser Ser Val Lys Ser Val Pro Glu Asn Leu
                   85                  90                  95
   ctc aag tgc ttt gga gaa cag aat aaa aaa cag ccc cgc caa gac tat       336
   Leu Lys Cys Phe Gly Glu Gln Asn Lys Lys Gln Pro Arg Gln Asp Tyr
               100                 105                 110
   cag ctg gga ttc act tta att tgg aag aat gtg ccg aag acg cag atg       384
   Gln Leu Gly Phe Thr Leu Ile Trp Lys Asn Val Pro Lys Thr Gln Met
           115                 120                 125
   aaa ttc agc atc cag acg atg tgc ccc atc gaa ggc gaa ggg aac att        432
   Lys Phe Ser Ile Gln Thr Met Cys Pro Ile Glu Gly Glu Gly Asn Ile
       130                 135                 140
   gca cgt ttc ttg ttc tct ctg ttt ggc cag aag cat aat gct gtc aac        480
   Ala Arg Phe Leu Phe Ser Leu Phe Gly Gln Lys His Asn Ala Val Asn
   145                 150                 155                 160
   gca acc ctt ata gat agc tgg gta gat att gcg att ttt cag tta aaa        528
   Ala Thr Leu Ile Asp Ser Trp Val Asp Ile Ala Ile Phe Gln Leu Lys
                   165                 170                 175
   gag gga agc agt aaa gaa aaa gcc gct gtt ttc cgc tcc atg aac tct        576
   Glu Gly Ser Ser Lys Glu Lys Ala Ala Val Phe Arg Ser Met Asn Ser
               180                 185                 190
   gct ctt ggg aag agc cct tgg ctc gct ggg aat gaa ctc acc gta gca        624
   Ala Leu Gly Lys Ser Pro Trp Leu Ala Gly Asn Glu Leu Thr Val Ala
           195                 200                 205
   gac gtg gtg ctg tgg tct gta ctc cag cag atc gga ggc tgc agt gtg        672
   Asp Val Val Leu Trp Ser Val Leu Gln Gln Ile Gly Gly Cys Ser Val
       210                 215                 220
   aca gtg cca gcc aat gtg cag agg tgg atg agg tct tgt gaa aac ctg        720
   Thr Val Pro Ala Asn Val Gln Arg Trp Met Arg Ser Cys Glu Asn Leu
   225                 230                 235                 240
   gct cct ttt aac acg gcc ctc aag ctc ctt aag    tga                      756
   Ala Pro Phe Asn Thr Ala Leu Lys Leu Leu Lys
                   245                 250
<210> 2
   <211> 251
   <212> PRT
   <213> Homo sapiens
<400> 2
   Met Pro Met Tyr Gln Val Lys Pro Tyr His Gly Gly Gly Ala Pro Leu
    1               5                  10                  15
   Arg Val Glu Leu Pro Thr Cys Met Tyr Arg Leu Pro Asn Val His Gly
               20                  25                  30
   Arg Ser Tyr Gly Pro Ala Pro Gly Ala Gly His Val Gln Asp Tyr Gly
           35                  40                  45
   Ala Leu Lys Asp Ile Val Ile Asn Ala Asn Pro Ala Ser Pro Pro Leu
       50                  55                  60
   Ser Leu Leu Val Leu His Arg Leu Leu Cys Glu His Phe Arg Val Leu
   65                  70                  75                  80
   Ser Thr Val His Thr His Ser Ser Val Lys Ser Val Pro Glu Asn Leu
                   85                  90                  95
   Leu Lys Cys Phe Gly Glu Gln Asn Lys Lys Gln Pro Arg Gln Asp Tyr
               100                 105                 110
   Gln Leu Gly Phe Thr Leu Ile Trp Lys Asn Val Pro Lys Thr Gln Met
           115                 120                 125
   Lys Phe Ser Ile Gln Thr Met Cys Pro Ile Glu Gly Glu Gly Asn Ile
       130                 135                 140
   Ala Arg Phe Leu Phe Ser Leu Phe Gly Gln Lys His Asn Ala Val Asn
   145                 150                 155                 160
   Ala Thr Leu Ile Asp Ser Trp Val Asp Ile Ala Ile Phe Gln Leu Lys
                   165                 170                 175
   Glu Gly Ser Ser Lys Glu Lys Ala Ala Val Phe Arg Ser Met Asn Ser
               180                 185                 190
   Ala Leu Gly Lys Ser Pro Trp Leu Ala Gly Asn Glu Leu Thr Val Ala
           195                 200                 205
   Asp Val Val Leu Trp Ser Val Leu Gln Gln Ile Gly Gly Cys Ser Val
       210                 215                 220
   Thr Val Pro Ala Asn Val Gln Arg Trp Met Arg Ser Cys Glu Asn Leu
   225                 230                 235                 240
   Ala Pro Phe Asn Thr Ala Leu Lys Leu Leu Lys
                   245                 250
<210> 3
   <211> 20
   <212> DNA
   <213> Artificial Sequence
<220>
   <223> primer 6
<400> 3
   agtgctttgg agaacagaat 20
<210> 4
   <211> 20
   <212> DNA
   <213> Artificial Sequence
<220>
   <223> primer 7
<400> 4
   aagagcagag ttcatggagc 20
<210> 5
   <211> 20
   <212> DNA
   <213> Artificial Sequence
<220>
   <223> primer 1
<400> 5
   tctgacggtt tctgagcgtt 20
<210> 6
   <211> 20
   <212> DNA
   <213> Artificial Sequence
<220>
   <223> primer 5
<400> 6
   aagtgaatcc cagctgatag 20
<210> 7
   <211> 21
   <212> DNA
   <213> Artificial Sequence
<220>
   <223> primer DX2-F
<400> 7
   tgctttggtt ctgccatgcc g 21
<210> 8
   <211> 21
   <212> DNA
   <213> Artificial Sequence
<220>
   <223> primer DX2-B
<400> 8
   cgtaatcctg cacgtggcca g 21
<210> 9
   <211> 54
   <212> DNA
   <213> Artificial Sequence
<220>
   <223> si-DX2 #3 coding nucleic acid sequence
<400> 9
   tcgaggccac gtgcaggtta cgggagtagg ccgtaatcct gcacgtggcc tttt 54
<210> 10
   <211> 55
   <212> DNA
   <213> Artificial Sequence
<220>
   <223> si-DX2 #3 coding nucleic acid sequence
<400> 10
   ctagaaaagg ccacgtgcag gattacggca gactcccgta atcctgcacg tggcc 55
<210> 11
   <211> 58
   <212> DNA
   <213> Artificial Sequence
<220>
   <223> si-DX2 #4 coding nucleic acid sequence
<400> 11
   tcgagctggc cacgtgcagg attacgagta ctggtaatcc tgcacgtggc cagctttt 58
<210> 12
   <211> 58
   <212> DNA
   <213> Artificial Sequence
<220>
   <223> si-DX2 #4 coding nucleic acid sequence
<400> 12
   ctagaaaagc tggccacgtg caggattacc agtactcgta atcctgcacg tggccagc 58
<210> 13
   <211> 56
   <212> DNA
   <213> Artificial Sequence
<220>
   <223> si-DX2 #5 coding nucleic acid sequence
<400> 13
   tcgacacgtg caggattacg gggcgagtac tggccccgta atcctgcacg tgtttt 56
<210> 14
   <211> 56
   <212> DNA
   <213> Artificial Sequence
<220>
   <223> si-DX2 #5 coding nucleic acid sequence
<400> 14
   ctagaaaaca cgtgcaggat tacggggcca gtactcgccc cgtaatcctg cacgtg 56
<210> 15
   <211> 15
   <212> DNA
   <213> Artificial Sequence
<220>
   <223> RT primer
<400> 15
   cagcaccacg tctgc 15
<210> 16
   <211> 24
   <212> DNA
   <213> Artificial Sequence
<220>
   <223> primer DX2-S2
<400> 16
   ctggccacgt gcaggattac gggg 24
<210> 17
   <211> 30
   <212> DNA
   <213> Artificial Sequence
<220>
   <223> primer f3
<400> 17
   gagcatctga gtttgacccc tggaatctac 30
<210> 18
   <211> 35
   <212> DNA
   <213> Artificial Sequence
<220>
   <223> priemr r2
<400> 18
   ttgcaggatg ttggttacgt ccaagtctgc atctg 35
<210> 19
   <211> 31
   <212> DNA
   <213> Artificial Sequence
<220>
   <223> priemr f6
<400> 19
   gtcaccgaat gtaactgtgc tggcttcgaa g 31
<210> 20
   <211> 30
   <212> DNA
   <213> primer pKOf2
<400> 20
   ggtaggtcca gagtcttcag agatcaagtc 30
<210> 21
   <211> 30
   <212> DNA
   <213> primer r6
<400> 21
   ccataaccca ggttgacggg tttagtgccc 30